# EUROPEAN PATENT APPLICATION

(11) **EP 4 311 862 A1**
(43) Date of publication of application: **31.01.2024**
(21) Application number: 22382728.8
(22) Date of filing: 29.07.2022
(51) Int. Cl.: C12Q 1/6883

(54) **METHODS FOR DETECTION OF EMBRYO IMPLANTATION FAILURE OF ENDOMETRIAL ORIGEN**

(71) Applicant: IVI RMA GLOBAL, SL., 46004 Valencia (ES)
(72) Inventor: Díaz Gimeno, Patricia, 46111 Rocafort - Valencia (ES); Sebastián León, Patricia, 46014 Vaencia (ES); Pellicer Martínez, Antonio, 00197 Rome (ES)
(74) Representative: Carlos Hernando, Borja

(57) **Abstract**

The present invention is based, in part, on the definition of a plurality of panels of genes and/or fragments thereof (target sequences within the genes) as defined in the claims, which allow the accurate classifying of an endometrium being at risk of having IF, including RIF. The combinations of genes and/or fragments thereof used according to the present invention result in a surprising improvement in the ability to classify an endometrium being in a pathological state and therefore being at risk of having an IF irrespective of progression of endometrial dating. The invention discloses an in vitro method for prognosing the risk of implantation failure of an embryo in a subject, wherein said implantation failure is due to molecular disruption of endometrium, (i.e., pathological endometrium) thereby excluding implantation failure due to menstrual cycle progression variations in the timing of the window of implantation (WOI) . This has been achieved by defining specific gene panels from an endometrial pathology gene panel where gene expression data corresponding to variations in the timing of the WOI were removed.

## Description

### TECHNICAL FIELD

This invention relates generally to methods, and systems for identifying biomarkers for prognosing risk of endometrial infertility, in particular, prognosing implantation failure (IF) of endometrial origin in an embryo in the endometrium of a subject.

### BACKGROUND

The breakthrough of assisted reproductive technology (ART) in recent scientific history has allowed couples previously unable to conceive to achieve viable pregnancy, while at the same time opening a new window into detection of early miscarriages. Although ART has improved outcomes for struggling couples, a new challenge has emerged: recurrent implantation failure (RIF). Multiple failed cycles can leave couples frustrated and desperate for explanations. It is necessary to determine the etiologies of RIF in order to propose new and beneficial solutions for these patients.

Patients who experience repeated instances of failed implantation receive a diagnosis of recurrent implantation failure (RIF). While there are more than 76 clinical definitions of RIF (Polanski et al., 2014), the condition typically is diagnosed as a symptom after three or more implantation failures with good-quality embryos from ovum donation or euploid embryos tested genetically (Coughlan et al., 2014). The prevalence of RIF remains unclear (Busnelli et al., 2020). For example, evaluation in patients of implantation rate per transfers of euploid embryos diagnosed by genetic screening indicated an average implantation rate per transfer of only 63.3% (Pirtea et al., 2021). Further, RIF has a heterogeneous etiology that confounds its diagnosis, clinical definition, and even research studies (Coughlan et al., 2014; Craciunas et al., 2019; Polanski et al., 2014). Clinical symptoms do not provide sufficient criteria to stratify patients because of challenges in confirming the number of previous failed cycles with good-quality embryos, especially because RIF is sometimes defined considering only those implantation failures registered within the same clinic. These issues may result in patient misclassification in early stages of treatment since the threshold is that they must have experienced 3 or more failed implantations (Sun et al., 2021). New strategies are needed to identify potential endometrial causes that could lead to RIF, to guide treatment approaches that improve pregnancy rates at the first transfer attempt.

Precision medicine approaches provide already means to advance reproductive success in assisted reproduction treatments (ARTs). One key factor for reproductive success is the status of the maternal endometrium that supports embryo implantation. In ARTs, specifically in vitro fertilization (IVF), the endometrium underlies an estimated two-thirds of implantation failures (Craciunas et al., 2019).

The human endometrium is a dynamic tissue that undergoes molecular and morphological modifications throughout the menstrual cycle (Sandra, 2016). Under the control of oestrogen and progesterone, these tissue dynamics ultimately promote a state of receptivity that enables embryo implantation. This short period of the mid-secretory phase is called the window of implantation (WOI) (Wilcox et al., 1999). The WOI and other endometrial phases are commonly assessed during both reproductive biomedical research (Devesa-Peiro et al., 2021) and IVF by endometrial dating (Diaz-Gimeno et al., 2014).

Classically, the WOI was estimated by determining menstrual cycle days, ovulation timing and hormone levels. More precise endometrial dating relied on histological methods as the gold standard (Noyes et al., 1975). However, histological assessment is subjective and therefore may offer limited accuracy and reproducibility (Coutifaris et al., 2004; Murray et al., 2004). In addition, endometrial transcriptomics has proven more objective and concordant with endometrial progression for endometrial tissue dating than pathologists when compared to the LH peak (Diaz-Gimeno et al., 2011). Although other approaches have included ultrasound and identifying single molecular biomarkers (Craciunas et al., 2019), transcriptomics additionally offers deeper molecular information that enables distinguishing new patterns of disease or processes and thereby providing new taxonomies (Diaz-Gimeno et al., 2017; Mirnezami et al., 2012; Sebastian-Leon et al., 2018).

Transcriptomics has been applied for identifying molecular biomarkers of cycle progression in healthy women (Altmäe et al., 2017; Bhagwat et al., 2013; Borthwick et al., 2003; Carson et al., 2002; Diaz-Gimeno et al., 2011; Haouzi et al., 2009; Kao, 2002; Mirkin et al., 2005; Ponnampalam et al., 2004; Punyadeera et al., 2005; Riesewijk et al., 2003; Sigurgeirsson et al., 2017; Talbi et al., 2006) and women with infertility undergoing IVF (Altmäe et al., 2009; Bersinger et al., 2008; Koler et al., 2009; Koot et al., 2016; Shi et al., 2018; Tapia et al., 2008).

As such, several patent documents disclose methods for prognosing embryo implantation success in a subject based on determining transcriptomic profiles associated with the window of implantation phase.

US 10,081,840B2 discloses a molecular diagnostic tool allowing analyzing the transcriptome of a subset of 238 genes of the genome related to the endometrial receptivity status, where "endometrial receptivity status" is understood as the state in which the endometrium is prepared for embryo implantation, thus taking place in all menstrual cycles in a time period, thus during WOI.

US 9,260,748B2 describes a method of assessing if the endometrium of a subject is receptive, which is based in identifying genes expressed in human endometrium during the WOI, comprising the steps of: a) obtaining an endometrial biopsy sample from a patient; b) measuring, in said endometrial biopsy sample, the expression level of each of the eleven genes MFAP5, ANGPTLI, PROK1, NLF2, LAMB3, BCL2L10, 15 CD68, TRPC4, SORCSI, FST and KRTS0; c) comparing the expression level of each of the eleven genes in the endometrial biopsy sample with control expression levels of the eleven genes from endometrial biopsy sample obtained from a non-receptive endometrium; and d) assessing the endometrium of the patient as being receptive when all of said eleven genes are overexpressed compared to the control.

US 10,918,327 B2 claims a method of predicting endometrial receptivity status for embryonic implantation in a human subject, the method comprising: (a) providing a first biological sample obtained from a human subject at a first time point within a menstrual cycle; 5 10 15 20 25 30 35 40 45 50 55 60 65 38; (b) determining a gene expression profile of a panel of genes in the first biological sample, wherein the panel of genes consists of: Annexin A4 (ANXA4), Cation channel sperm auxiliary subunit beta (CATSPERB), Prostaglandin F receptor (PTGFR), Prostaglandin-endoperoxide synthase 1 (prostaglandin G/H synthase and cyclooxygenase) (PTGS1), Interleukin-8 (IL8), Secretoglobin, family 2A, member 2 (SCGB2A2), Angiopoietin-like 1 (ANGPTL1), Hypoxanthine phosphoribosyltransferase 1 (HPRT1), Matrix metallopeptidase 10 (MMP10), Progesterone Receptor (PGR), Integrin alpha 8 (ITGA8), Interferon gamma (IFNG), Prokineticin-1 (PROK1), Forkhead box protein O1 (FOXO1), C-X-C motif chemokine ligand 1 (CXCL1), Stanniocalcin-1 (STC1), Matrix Metallopeptidase 9 (MMP9), Mucin 1 (MUC1), Ribosomal protein L13a (RPL13A), Calcitonin-related polypeptide alpha (CALCA), Integrin subunit alpha-9 (ITGA9), Rac GTPase-activating protein 1 (RACGAP1), Glutathione peroxidase 3 (GPX3), Protein phosphatase 2, regulatory subunit B, gamma (PPP2R2C), Arginase 2 (ARG2), Secretoglobin, family 3A, member 1 (SCGB3A1), Aldehyde dehydrogenase family 1 member A3 (ALDH1A3), Apolipoprotein D (APOD), C2 calcium-dependent domain-containing protein 4B (C2CD4B), Trefoil factor 3 (TFF3), Aquaporin-3 (AQP3), Gap junction protein, alpha 4 (GJA4), Rho GDP-dissociation inhibitor alpha (ARHGDIA), Selectin L (SELL), Apolipoprotein L, 2 (APOL2), Metallothionein-1H (MT1H), Metallothionein-IX (MT1X), Metallothionein-IL (MT1L), Monoamine oxidase AA (MAOA) and Metallothionein-IF (MTIF) using reverse transcription polymerase chain reaction analysis; and (c) comparing the determined gene expression profile in step (b) with a gene expression profile of the same panel of genes listed in step (b) of a receptive endometrial receptivity reference group, a non-receptive endometrial receptivity reference group, a pre-receptive endometrial receptivity reference group, and a post-receptive endometrial receptivity reference group; (d) identifying the human subject as having: i) a receptive endometrial status, wherein the determined gene expression profile corresponds to a gene expression profile of the panel of genes of a receptive endometrial receptivity reference group, (ii) a non-receptive endometrial status, wherein the determined gene expression profile corresponds to a gene expression profile of the panel of genes of a non-receptive endometrial receptivity reference group, (iii) a pre-receptive endometrial status, wherein the determined gene expression profile corresponds to a gene expression profile of the panel of genes of a pre-receptive endometrial receptivity reference group, or (iv) a post-receptive endometrial status, wherein the determined gene expression profile corresponds to a gene expression profile of the panel of genes of a post-receptive endometrial receptivity reference group; and (e) transferring a pre-implantation embryo into the human subject identified as having a receptive endometrial status.

EP3607095B2 is also based on identification of transcriptomic patterns related to the WOI and discloses a method for determining the receptivity status of an endometrium comprising the quantification of the expression of the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, in a sample.

WO2019246160 discloses also a method based on determining biomarker genes for the WOI, such as a method for detecting that a subject is within WOI, comprising: (a) determining a level of expression of at least twenty genes in a sample of endometrial cells obtained from a subject, wherein the twenty genes are selected from the group consisting of : PLUA, MMP7, THBS1, CADM1, NPAS3, ATP1A1, ANK3, ALPL, TRAK1, SCGB1D2, MT1F, MT1X, MT1E, MT1G, CXCL14, MAOA, DPP4, NUPR1, GPX3, and PAEP; (b) comparing the determined level of expression of each of the at least twenty genes with a control level; and (c) determining whether the subject is within the WOI, wherein the subject is identified as being within the WOI if the level of the expression of at least twenty genes is at least two-fold higher than a control level.

US 2019/0241955 is also based in identifying the window of implantation and describes a method of predicting endometrial receptivity for embryonic implantation in a human subject, the method comprising:
(a) providing a first biological sample obtained from a human subject at a first time point within a menstrual cycle; (b) determining the gene expression profile of a panel of genes in the first biological sample, wherein the panel of genes comprises two or more genes selected from the group consisting of: Annexin A4 (ANXA4), Apolipoprotein D (APOD), Apolipoprotein L2 (APOL2), Aquaporin-3 (AQP3), Amphiregulin (AREG), Arginase-2 (ARG2), ATP synthase FI subunit beta (ATP5F1B), Calpain-6 (CAPN6), Corepressor interacting with RBPJ (CIR1), CKLF-like MARVEL transmembrane domain-containing protein 5 (CMTM5), Catenin alpha 2 (CTNNA2), C-X-C motif chemokine ligand 1 (CXCL1), C-X-C motifchemokine ligand 6 (CXCL6), Estrogen receptor 1 (ESRI), Forkhead box P3 (FOXP3), Growth arrest and DNA damage-inducible alpha (GADD45A), Gap junction protein alpha 4 (GJA4), Glutathione peroxidase 3 (GPX3), Hemoglobin subunit alpha (HBA1), Hemoglobin subunit gamma-1 (HBG1), Hydroxymethylbilane synthase (HMBS), Homeobox B7 (HOXB7), Insulin-like growth factor-binding protein 1 (IGFBP1), Interleukin-1 Receptor type 1 (IL1R1), Interleukin-4 (IL4), Interleukin-5 (IL5), Integrin subunit alpha-9 (ITGA9), Integrin beta-1 (ITGB1), Monoamine oxidase A (MAOA), Mitogen-activated protein kinase 1 (MAPK1), Matrix Metallopeptidase 9 (MMP9), Metallothionein-IE (MT1E), Metallothionein-IF (MT1F), Metallothionein-1H (MT1H), Metallothionein-1L (MT1L), Metallothionein-Ix (MT1X), NF-kappa-B inhibitor alpha (NFKBIA), Nicotinamide N-methyltransferase (NMMT), Progesterone Receptor (PGR), progesterone receptor membrane component 1 (PGRMC1), phospholipaseA2 group IVA (PLA2G4A), Prostaglandin-endoperoxidase synthase 1 (PTGS1), Ras homolog family member A (RHOA), ribosomal protein L13a (RPL13A), Secretoglobin family 2A member 2 (SCGB2A2), Succinate dehydrogenase complex flavoprotein subunit A (SDHA), Serpin family A member 1 (SERPINA1), and Trefoil factor 3 (TFF3) using quantitative real-time polymerase chain reaction (real-time qPCR) analysis; and (c) identifying the human subject as having: (i) a receptive endometrial status, when the determined gene expression profile corresponds to a gene expression profile of the panel of genes of a receptive endometrial receptivity reference group, (ii) a non-receptive endometrial status, when the determined gene expression profile corresponds to a gene expression profile of the panel of genes of a non receptive endometrial receptivity reference group, (iii) a pre-receptive endometrial status, when the determined gene expression profile corresponds to a gene expression profile of the panel of genes of a pre receptive endometrial receptivity reference group, or (iv) a post-receptive endometrial status, when the determined gene expression profile corresponds to a gene expression profile of the panel of genes of a post receptive endometrial receptivity reference group.

New strategies to identify RIF and other conditions are possible using high-throughput technologies. For example, many transcriptomic studies have characterized both healthy endometrium and endometrial pathology by evaluating endometrial gene expression at different menstrual cycle stages (Altmäe et al., 2017; Bhagwat et al., 2013; Borthwick et al., 2003; Carson et al., 2002; Diaz-Gimeno et al., 2022; Diaz-Gimeno et al., 2011; Haouzi et al., 2009; Kao, 2002; Mirkin et al., 2005; Ponnampalam et al., 2004; Punyadeera et al., 2005; Riesewijk et al., 2003; Sebastian-Leon et al., 2021; Talbi et al., 2006) or by comparing patients with RIF to controls using clinical criteria in the mid-secretory phase (Altmäe et al., 2009; Bastu et al., 2019; Bersinger et al., 2008; Koler et al., 2009; Pathare et al., 2017; Shi et al., 2018; Tapia et al., 2008). Notably, however, most studies using clinical criteria for RIF retrieved no or few differentially expressed genes (DEGs) compared to controls(Devesa-Peiro et al., 2021; Koot et al., 2016), likely reflecting the molecular heterogeneity of this condition by clinical criteria. Yet, one study of RIF that retrieved informative DEGs was really measuring the endometrial progression effect instead of the genes associated with the condition of interest (Devesa-Peiro et al., 2021), as referred in the above cited patent documents.

However, on top of the difficulty parsing the molecular heterogeneity in clinical taxonomy, the menstrual cycle progression also masks endometrial pathology as a cause for IF in a subject-the progression bias has stronger effect on the transcriptome than any potential pathology affecting endometrial function. While detecting endometrial progression for endometrial dating is widely applied as a diagnostic tool in the clinical setting (Altmäe et al., 2017; Diaz-Gimeno et al., 2022; Diaz-Gimeno et al., 2011; Enciso et al., 2018), there is no method for diagnosing transcriptomic disruption or pathology independent of menstrual cycle progression variations in the WOI.

Consequently, the authors of the present disclosure have reported the need to correct for the menstrual cycle progression variations effect in transcriptomic analyses to detect endometrial pathology as a primary cause for determining the risk of endometrial infertility, more precisely, the risk of IF, including RIF.

Thus, the present invention is faced with the problem of providing a more reliable and effective in vitro method of diagnosing endometrial infertility, more precisely prognosing the risk of IF of an embryo in the endometrium of a subject, including recurrent implantation failure (RIF), which is based on detecting an endometrium pathology state as the primary cause for IF. As it will be shown in the following disclosure the present method provides for a more effective method than a method identifying endometrial infertility based on transcriptomics identifying the menstrual cycle progression variations.

Thus, the present disclosure describes for the first time an effective and more reliable method of diagnosing endometrium infertility or prognosing in a subject risk of IF including RIF, which is based on identifying transcriptomics associated to an endometrium which is in a pathologic state, thus an endometrium with poor prognosis for embryo implantation.

### SUMMARY

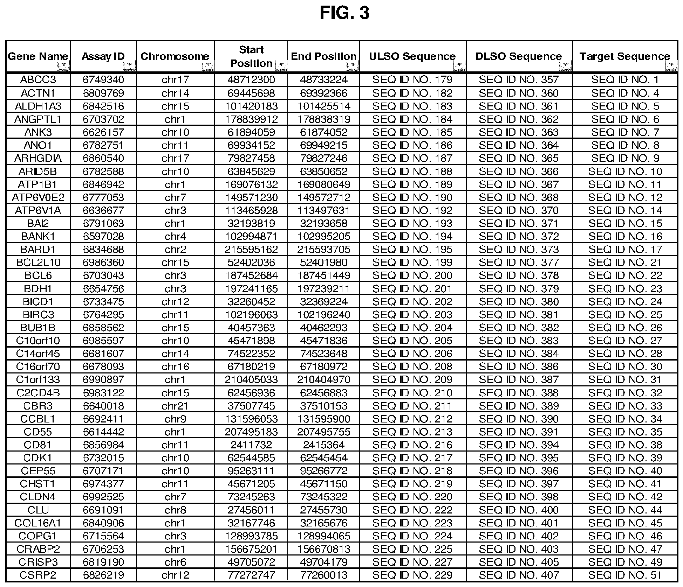

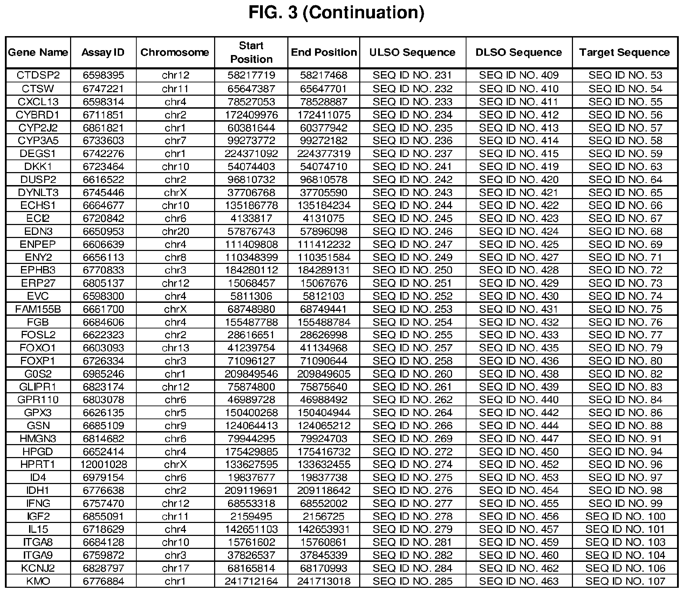

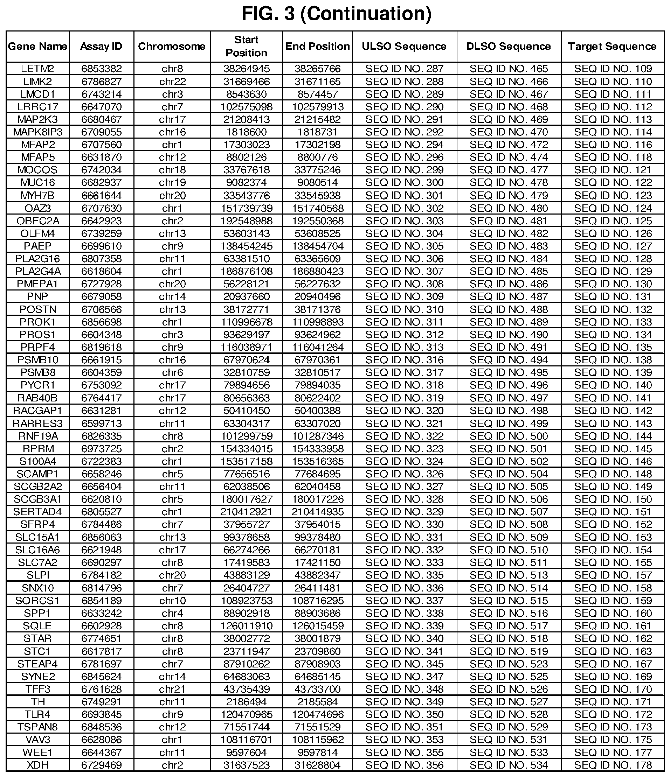

The present disclosure is defined in the appended claims. The present disclosure relates to biomarkers and methods for prognosing the risk of IF of an embryo into the endometrium of a subject, in particular a primate or a human female subject. Biomarkers used in the method herein disclosed are genes and corresponding fragments (target sequences) thereof as listed in Figure 3.

In certain embodiments, the present invention provides in vitro methods for prognosing the risk of IF of an embryo into the endometrium of a subject, in particular a primate or a human female subject, comprising measuring in a biological sample isolated from said subject, the level of expression of at least a plurality of genes and/or fragments thereof selected from Figure 3; comparing weather such genes are either expressed or differentially expressed with respect to a reference healthy control sample, and determining risk of implantation failure in the subject if each of these genes and/or fragments thereof are either expressed, over-expressed or under-expressed when compared to the reference control sample.

Advantageously the above disclosed method is capable of prognosing IF due to the endometrium being in a pathological state and disregards considering gene expression data associated to genes reflecting a menstrual cycle progression effect. Thus, avoiding the masking of gene expression data associated to endometrial pathology leading to implantation failure.

The disclosure also provides a kit to be used according to the aforementioned methods for prognosing IF, which comprises probes for measuring the expression level of the genes and/or fragments thereof, all of which are disclosed in Figure 3.

Unless otherwise defined, all technical and scientific terms used herein have the same meaning as commonly understood by one of ordinary skill in the art to which this invention pertains. Although methods and materials similar or equivalent to those described herein can be used in the practice of the present disclosure, suitable methods and materials are described below.

In addition, the materials, methods and examples described herein are illustrative only and are not intended to be limiting. Features and advantages of the invention will be apparent from the following detailed description and claims.

### BRIEF DESCRIPTION OF THE DRAWINGS

The foregoing and other objects, aspects, features, and advantages of the present disclosure will become more apparent and better understood by referring to the following description taken in conjunction with the accompanying drawings, in which:
Figure 1 shows the results of removing the transcriptomic variations due to the menstrual cycle progression effect that mask the effect of the endometrial pathology. Subjects grouped by menstrual phase (A) will be grouped by Poor / Good prognosis after menstrual cycle effect is removed (B). (C) The menstrual cycle effect was removed from gene expression by keeping pathology effect.

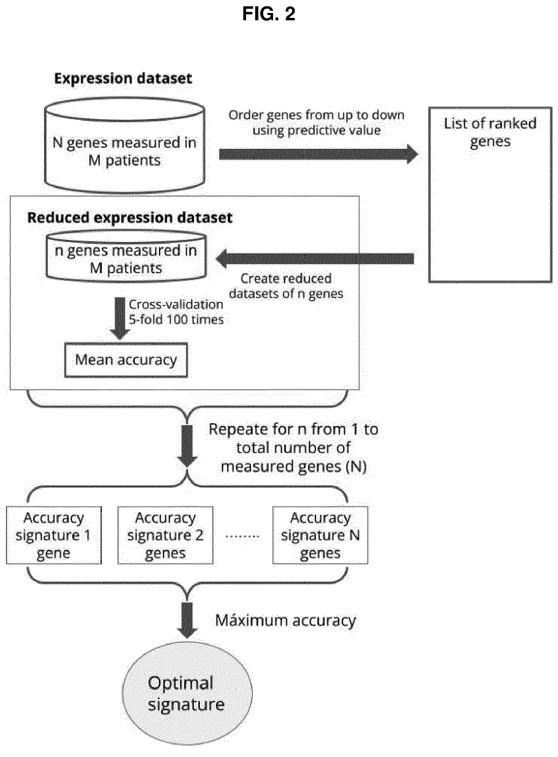

Figure 2 shows procedures to gene signature discovery. First, genes were ranked by predictive value calculated using the Correlation Attribute algorithm included in Weka. The sets of different sizes or ordered genes were performed by increasing size 1-by-1. For each subset size, n, the n better genes (higher Informative Score (IS) value, i.e. the most predictive genes), were selected. Then 100 prediction models were run using the reduced dataset that includes all samples and the subset of m selected genes, and prediction performance (accuracy) for these 100 models were calculated for each model using 5-fold cross-validation. These 100 accuracies were considered the distribution of accuracies for size n, and its mean was considered as the accuracy of the n better genes in our experiment. Finally, gene set size that retrieve the maximum accuracy was considered as the optimal gene set size and the gene composing of this set was considered the optimal signature.
Figure 3 lists the genes and fragments thereof used as biomarkers in the present disclosure. It includes the gene name, chromosome number (chr), the start ("start") and end ("end") positions of the relevant regions (fragments of the genes) on the chromosome, as well as the Upstream Locus-Specific Oligo (ULSO sequence) and Downstream Locus-Specific Oligo, (DLSO sequence), which are the probes for targeting the target sequences in the method for determining expression levels thereof.
Figure 4 lists the genes and fragments thereof named as ED signature and associated with WOI. It includes the gene name, chromosome number (chr), the start ("start") and end ("end") positions of the relevant regions (fragments of the genes) on the chromosome, as well as the Upstream Locus-Specific Oligo (ULSO sequence) and Downstream Locus-Specific Oligo, (UDLSO sequence), which are the probes for targeting the target sequences in the method for determining expression levels thereof.
Figure 5 illustrates the RNA seq experimental procedure followed: from the biological sample to the gene expression.
Figure 6 illustrates the methodology for obtaining a prediction model for stratifying subjects as being at a risk of implantation failure of an embryo in the endometrium, as detailed in example 1.
Figure 7 illustrates Clinical Acute Transcriptomics Stratification (CATranS) algorithm.
Figure 8 shows CATranS classification results and evaluation. **A.** Clinical vs CATranS classification of patients. **B.** Comparison of clinical outcomes at first transfer after biopsy. **C.** Cumulative pregnancy rate.
Figure 9 shows 5-fold cross-validation process.
Figure 10 lists results for functional analysis of the 122 differential expressed genes as listed in Table 1 and forming part of the panel of genes of Figure 3. Figure 10 shows for each established functional group: The name of the functional group; the number of functional terms (Gene Ontology (GO) and Kyoto Encyclopaedia of Genes and Genomes (KEGG)); and the number of down-regulated and up-regulated genes annotated on each functional group.
Figure 11 lists the genes and target sequences of the 404 genes of EP panel

### DETAILED DESCRIPTION

The present invention is based, in part, on the definition of a plurality of panels of genes and/or fragments thereof (target sequences within the genes) as defined in the claims, which allow the accurate classifying of an endometrium being at risk of having IF, including RIF. The combinations of genes and/or fragments thereof used according to the present invention result in a surprising improvement in the ability to classify an endometrium being in a pathological state and therefore being at risk of having an IF irrespective of progression of endometrial dating. Thus, compared to the use of individual genes or previously described gene combinations, the present invention provides superior diagnostic, prognostic and classifying results, to identify when an endometrium is at risk of suffering IF, including RIF. For example, the gene sets and methods of the present invention show better predictability and reliability than past models that were trained only on clinical classification and other models based on transcriptomic data related to the WOI, known from the state of the art or commercially available. With this new taxonomy of endometrial evaluation, the method disclosed in the present invention found that pathological endometrium had at least a 1.5, preferably, at least a 2.3, more preferably, a 3.3-times higher risk of implantation failure compared with healthy or good prognosis endometria.

Thus, the present invention discloses an in vitro method for prognosing the risk of implantation failure of an embryo in a subject, where said implantation failure is due to molecular disruption of endometrium, (i.e., pathological endometrium) thereby excluding implantation failure due to menstrual cycle progression variations in the WOI.

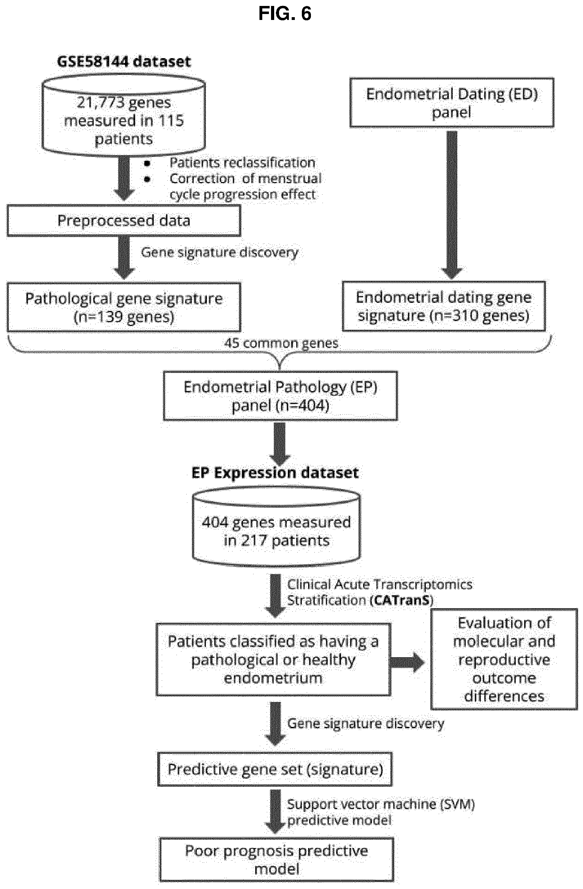

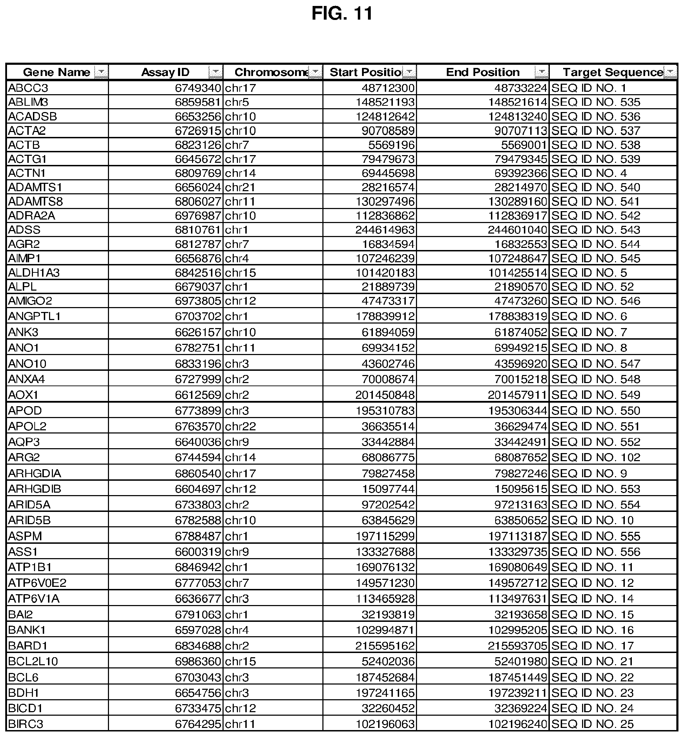

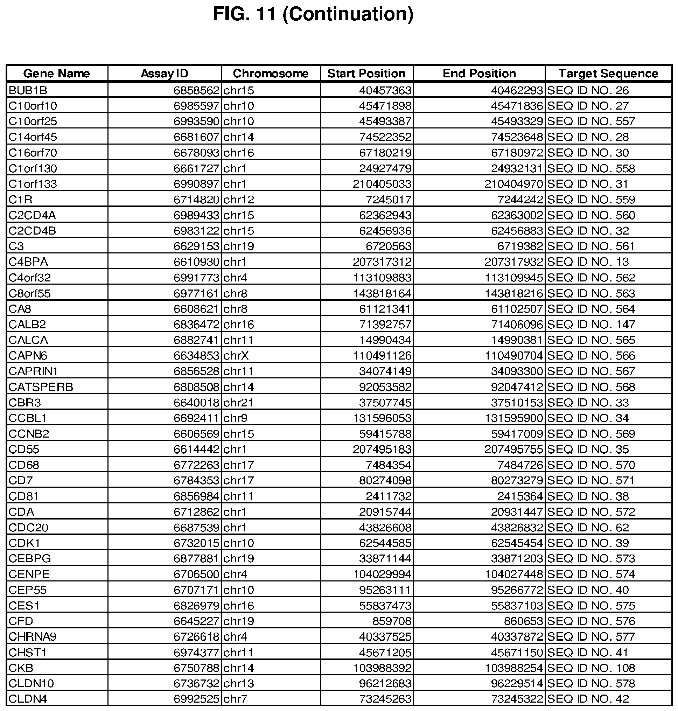

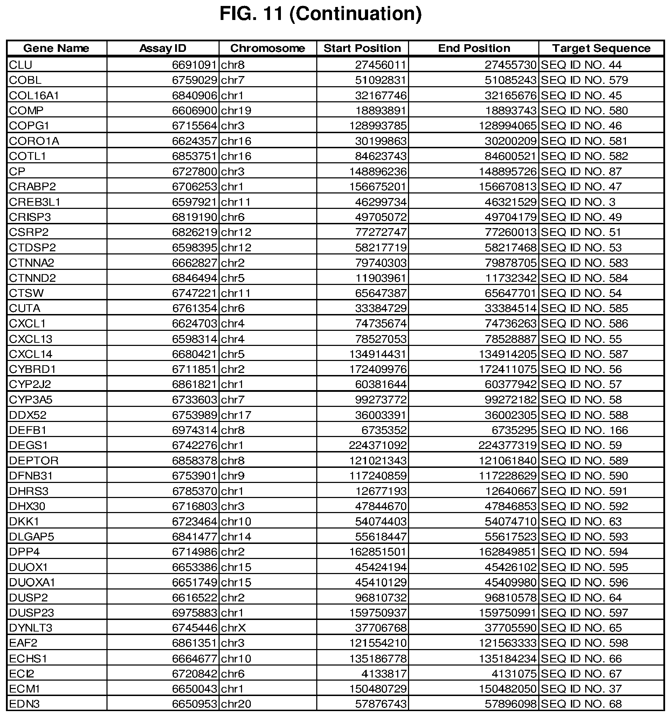

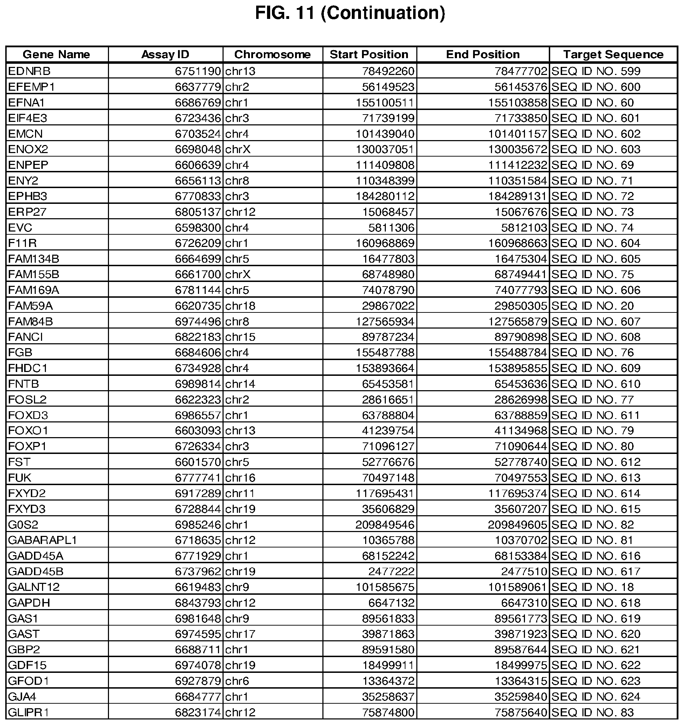

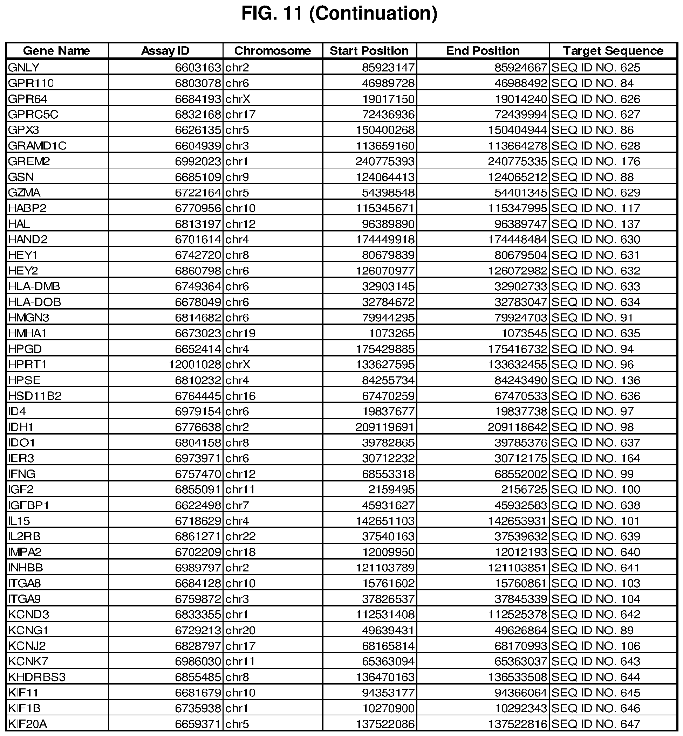

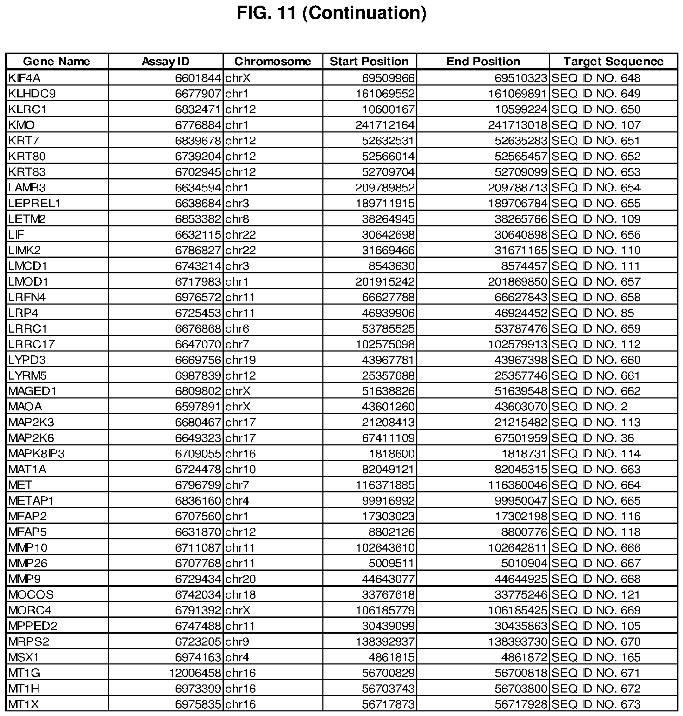

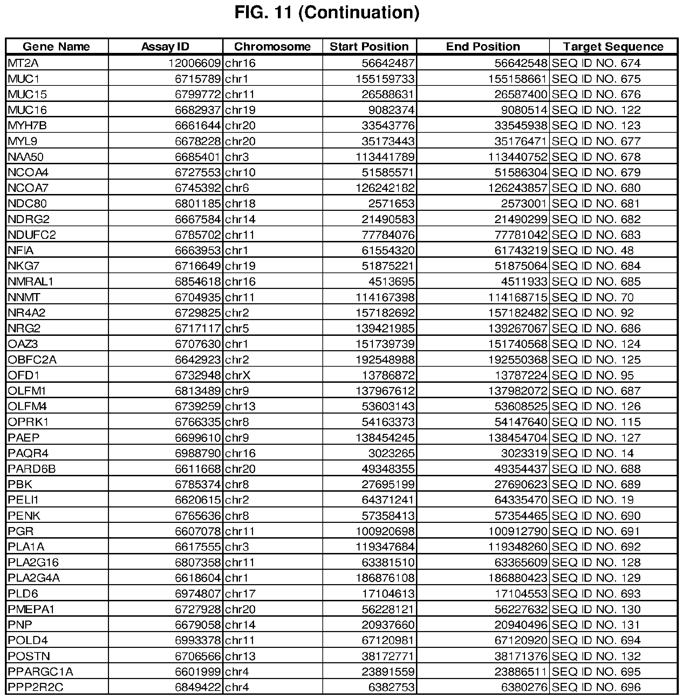

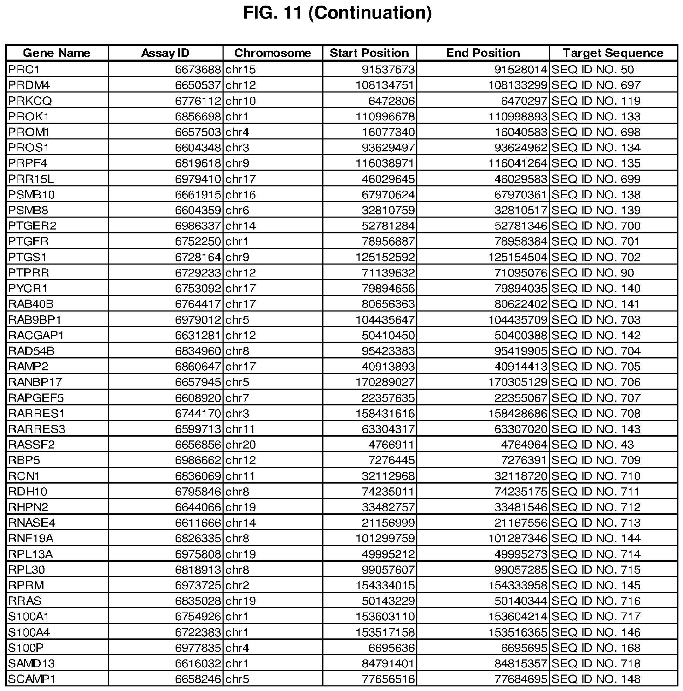

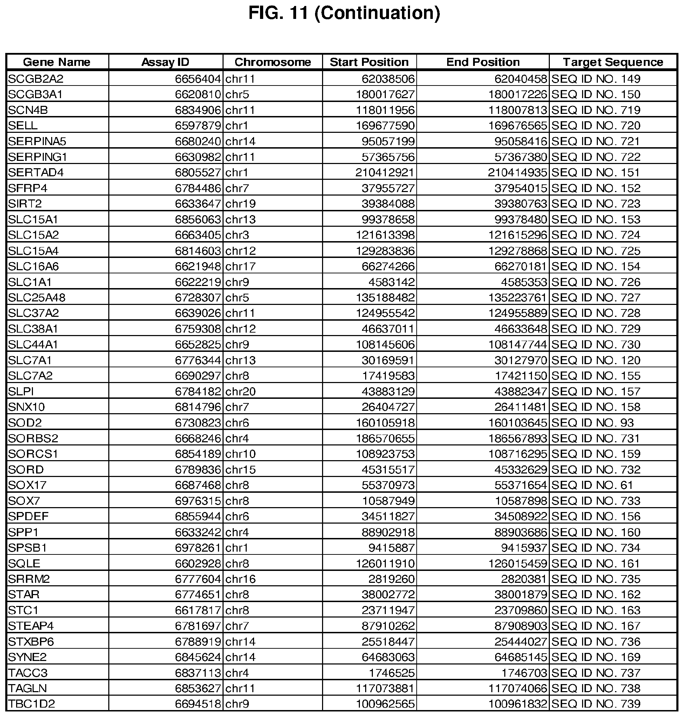

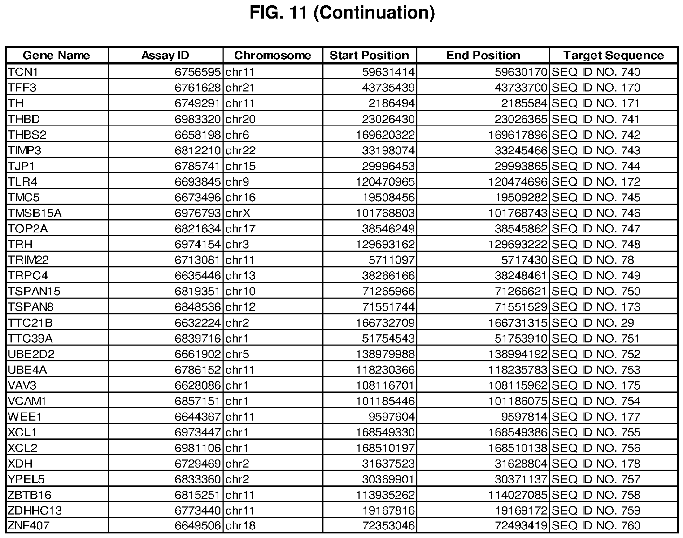

The present invention is based in the results obtained from the following study design as shown in Figure 6, where, as illustrated herein, genes to be included in the EP (Endometrial pathology) panel are selected from two different sources. First, the GSE58144 whole-transcriptome dataset was downloaded from Gene Expression Omnibus (GEO) and preprocessed. (Menstrual cycle progression effect based on an artificial intelligence (Al)-based taxonomy was corrected using linear models). Using the pre-processed data, a pathological gene signature discovery was performed (Figure 2) yielding a pathological signature comprising 139 genes. Additionally, 310 genes related to endometrial dating (ED) and encompassed in an "ED panel" as published in Diaz-Gimeno et al., Hum. Reprod. 2022, were added to the EP panel to properly correct endometrial displacement bias. The ED panel genes included pre-published gene-list related to endometrial dating (ERA^{®}, WIN^{®}, ERmap^{®} ) as well as an optimal signature of 50 genes that was selected from 4 different gene expression datasets from samples collected among the menstrual cycle (GSE98386, GSE29981, GSE58144 and GSE4888) according to their Informative score (IS), that means according to the capacity of these genes for classifying endometrial progression in each dataset (Figure 2). As a result of these gene selections, a 404 gene panel named as EP panel (Figure 11) was built and finally used for EP expression dataset, which comprises gene expression measures for 404 genes in 217 patients. These expression data were obtained using TruSeq Targets RNA-seq design and sequenced using Illumina Next seq 500 Sequencer. Next, patients were classified as having a pathological or healthy endometrium using a semi-supervised method called Clinical Acute Transcriptomics Stratification (CAtranS). Given this classification, a gene signature discovery process was applied to obtain a predictive gene set signature. Finally, this signature was used to train a Support Vector Machine predictive model to predict poor prognosis in the subjects

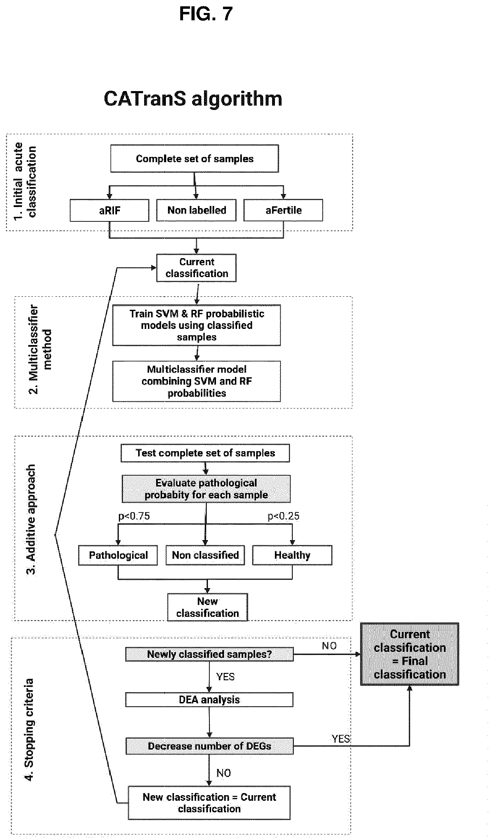

As illustrated in Figure 7, CATransS method includes, first, performing an initial acute classification of patients based on clinical parameters. This clinical acute classification defined participants as a patient with acute recurrent implantation failure (aRIF) or acute fertile (aFertile), and patients who did not fit the acute definition were classified as "non-labelled." Second, machine learning (SVM and RF) models were trained using only the labelled samples, defined as "current" classification, then classification probabilities obtained from each model were combined. The whole set of samples (including non-labelled) was tested to obtain the probability (p) of each sample having a pathological transcriptomic profile. According to this probability, each sample was newly classified as pathological if p>0.75, was newly classified as healthy if p<0.25, and was non-labelled otherwise. If any sample was reclassified into a different category, the new "current" category was considered the optimal classification according to transcriptomic profile. If any samples were reclassified, differential expression analysis (DEA) was performed between pathological and healthy groups, and the number of differentially expressed genes (DEGs) was evaluated. If the reclassification decreased the number of DEGs obtained with the previous classification, the previous "current" category was considered the optimal classification of samples according to their transcriptomic profile. For reclassified samples, a new combined machine learning model was trained and evaluated until the optimal classification was reached.

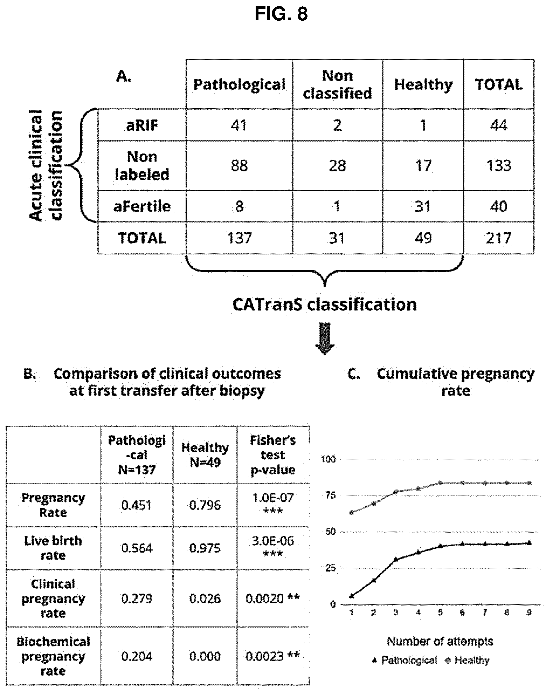

Figure 8 shows CATranS classification results and evaluation. **A.** Clinical vs CATranS classification of patients. Confusion matrix indicating how samples were classified in CATranS categories depending on their initial acute clinical classification. **B.** Comparison of clinical outcomes at first transfer after biopsy. Pregnancy rate (PR) was defined as the proportion of positive pregnancy tests obtained at the first transfer after biopsy in the patient population undergoing embryo transfer; live birth rate was defined as the number of ongoing gestations divided by the total number of positive pregnancy tests obtained at the first transfer after biopsy; clinical pregnancy rate was defined as the proportion of clinically recognized pregnancy loss before the 20th week of gestation related to the total number of pregnancies in which a gestational sac was visualized; and biochemical pregnancy rate was defined as the proportion of gestational losses after a positive pregnancy test without visualization of the gestational sac. Defined rates were represented as a bar per analyzed group. Differences in proportions were statistically tested using Fisher's exact test. Significant results are indicated with asterisks (*p<0.05, **p<0.01, ***p<0.001). **C.** Cumulative pregnancy rate. Using CATranS classification, the cumulative pregnancy rate associated with healthy or good prognosis endometrium was 63.3%% at first transfer and reached 83.7% at the fifth transfer, while the cumulative pregnancy rate associated with pathological or poor prognosis endometrium reached only 42.3% at the ninth transfer.

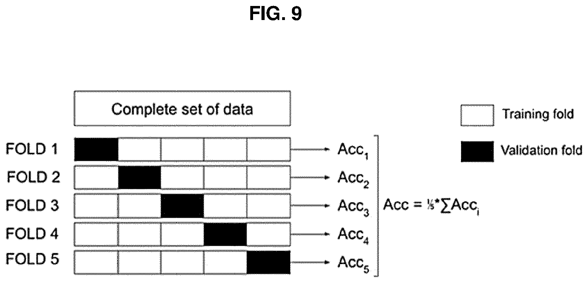

As shown in Figure 9, a 5-fold cross-validation process requires subjects being randomly divided in 5 sets and on each fold, four of the sets were used to train the model and the remaining set was used to validate the model and calculate the prediction performance of the model (accuracy) relative to each fold. Then the final accuracy of the cross-validation process is the mean of the accuracies obtained on each fold.

### Definitions:

As used herein, the term "biological sample" refers to a sample obtained or derived from a subject. The term "biological sample" encompasses a clinical sample, and also includes tissue obtained by surgical resection, tissue obtained by biopsy, cells in culture, cell supernatants, cell lysates, tissue samples, organs or organoids, bone marrow, blood, plasma, serum, endometrial fluid, exosome vesicles and the like. A "biological sample" includes a sample obtained from a subject's uterus, including embryo cultures. By way of example, the sample according to the present disclosure may be selected from a tissue, such as an endometrial tissue biopsy sample.

A "biopsy" refers to the process of removing a tissue sample for diagnostic or prognostic evaluation, and to the tissue specimen itself. Any biopsy technique known in the art can be applied to the obtention of an endometrial biopsy sample on which the prognostic methods of the present invention are preferably applied.

As used herein, "differential expression" refers to both quantitative as well as qualitative differences in the genes tissue expression patterns. Thus, a differentially expressed gene may have its expression activated (over- expressed) or inactive (under-expressed) in IF risk state versus normal endometrial conditions. Such a qualitatively regulated gene will exhibit an expression pattern within a given tissue that is detectable in either control or subjects with risk of IF or risk of RIF state. "Detectable", as used herein, refers to an RNA expression pattern that can be measured via the standard techniques. Generally, differential expression means at least a higher absolute fold change than |1|=(-1,+1) and this difference in expression must be statistically significant with an adjusted-p-value (False Discovery Rate (FDR))<0.05.

The terms "gene" and "gene product" or "expression product of a gene" are used interchangeably herein in reference to a gene, to refer to the RNA transcription products (transcripts) of the gene, including mRNA and the polypeptide translation products of such RNA transcripts, whether such product is modified post-translationally or not. A gene product can be, for example, an un-spliced RNA, an mRNA, a splice variant mRNA, a polypeptide, a post-translationally modified polypeptide, a splice variant polypeptide, etc.

As used herein the term "implantation failure (IF)" of endometrial origin refers to the absence of gestation after the transfer of good quality embryos in the blastocyst stage without obtaining gestation, i.e., a failed attempt to achieve a viable pregnancy by means of in vitro fertilization (IVF).

As used herein the term "recurrent implantation failure (RIF)" refers to the condition typically diagnosed as a symptom after three or more implantation failures with good-quality embryos from ovum donation or euploid embryos tested genetically.

The term "risk" as used herein with respect to prognosing IF, including RIF of an embryo in the endometrium of a subject refers to the qualitative of quantitative probability (whether expressed as a percentage or otherwise) that a particular subject will develop IF, i.e., the probability of not achieving full-term pregnancy in the next embryo transfer procedure when compared to a healthy subject. In some embodiments, e.g., as set forth herein, risk is expressed as a percentage. In some embodiments, e.g., as set forth herein, a risk is a qualitative of quantitative probability expressed in percentage that is equal to or greater than 0, 1, 2, 3, 4, 5, 6, 7, 8, 9, 10, 20, 30, 40, 50, 60, 70, 80, 90, or 100%. In some embodiments, e.g., as set forth herein, risk is expressed as a qualitative or quantitative level of risk relative to a reference risk or level or the risk of the same outcome attributed to a reference. In some embodiments, e.g., as set forth herein, relative risk is increased or decreased in comparison to the reference sample by a factor of 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 5, 6, 7, 8, 9, 10, or more.

As used herein, the term "subject" refers to primate or a human female subject. In particular, a human female subject. When the subject is a human, the subject can be genetically female (having XX sex chromosomes, or XXY sex chromosomes), premenopausal, and/or of advanced maternal age (e.g., over 35 years of age). For example, the human subject can have a history of miscarriages or stillbirths, a history of fertility issues/complications (e.g., pelvic inflammatory disease, endometriosis, polycystic ovarian syndrome, hormonal imbalances, premature ovarian aging/failure, antiphospholipid syndrome), and/or has previously had assisted reproductive treatments and/or hormone replacement therapies. In some examples, the human subject has had one or more cycles of IVF. In other examples, the subject has not had IVF coming from artificial insemination or timed intercourse. Subjects according to the present invention which were invited to participate in the experimental part of the present disclosure were scheduled for endometrial evaluation for medical decision or due to ≥2 implantation failures of idiopathic origin.

As used herein, the term "providing a prognosis" may refer to providing a prediction of the probable course and outcome of an embryo implantation capability in the endometrium of a subject. Providing a prognosis of an IF and RIF in a subject refers to providing a prediction of the subject being at risk of having an IF, including RIF.

The term "pathological endometrium" or "molecular disruption of endometrium" are used interchangeably herein in reference to subjects presenting a poor prognosis to outcome an embryo implantation in the following transfer after biopsy collection.

"Poor prognosis" or "poor endometrial prognosis" as used herein has the meaning of patients with an endometrium with higher risk of implantation failure, meaning that pathological endometrium has at least a 1.5, preferably, at least a 2.3, more preferably, a 3.3-times higher risk of implantation failure compared with healthy or good prognosis endometria.

"Good prognosis" or "good endometrial prognosis" as used herein has the meaning of patients with an endometrium with lower risk of implantation failure, meaning that that a healthy endometrium has at least a 1.5, preferably, at least a 2.3, more preferably, a 3.3-times lower risk of implantation failure compared with pathological or poor prognosis endometria.

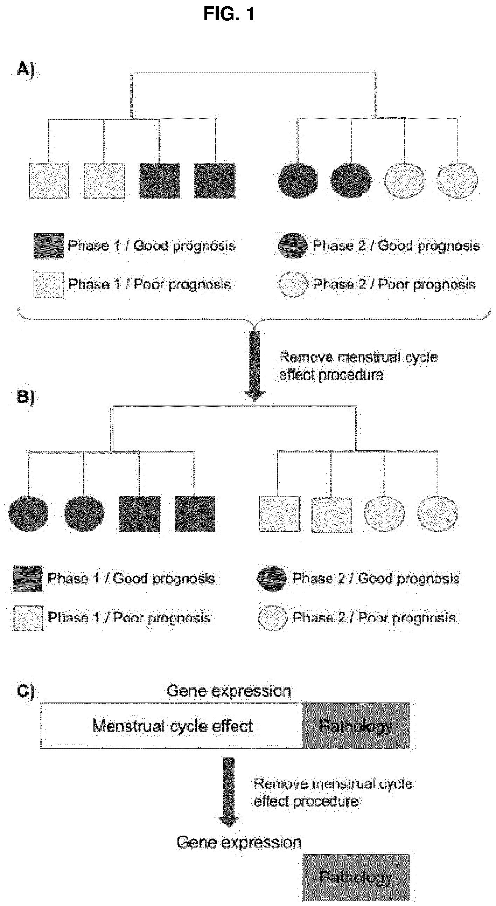

As used herein the term "remove menstrual cycle effect" refers to the mathematical procedure of removing from a gene data set the gene expression variability due to variations in endometrial progression in women's population while maintaining gene expression associated with the pathology of the endometrium. Briefly, the effect of the menstrual cycle is subtracted using linear models, resulting in a dataset only containing gene expression data related to poor or good endometrial prognosis due to existence of pathology (Figure 1). The expression of one gene could have a percentage of expression related to menstrual cycle progression and a percentage of expression related to the pathology. When we remove the gene expression variations related to menstrual cycle progression (menstrual cycle effect) the percentage related to the pathology is keeped and higlihgted (Figure1C).

As used herein "signature discovery" refers to the process to prioritize a subset of genes that allow to better predict the variable of interest (e.g. poor prognosis endometrium/risk of IF) and will be used in the following machine learning model to create a prediction model that correctly predicts the variable of interest. (Figure 2)

As used herein "ED model" refers to a machine learning model that allows to predict menstrual cycle phase from transcriptomic data measured on preferably, an endometrial biopsy from a subject. This model included a personalized gene panel including 310 genes selected by their relation to endometrial dating (ED panel), 73 of them were prioritized as ED signature used in the ED model.

In one embodiment the present invention is directed to an in vitro method of prognosing the risk of implantation failure of an embryo in a subject, said method further comprising determining the expression level of a plurality of genes and/or fragments thereof as well as their corresponding gene products in a biological sample isolated from the subject and comparing said expression level to a control gene expression profile known to be indicative of a healthy endometrium.

Genes and fragments thereof (target sequences) used for the purpose of the present invention are shown in Figure 3.

The authors of the present invention have found that prognosing the risk of implantation failure in a subject requires comparing the expression level of a plurality of genes and/or fragments thereof of Figure 3 in a biological sample isolated from a subject to a control (healthy) gene expression profile and determining that a risk of IF exists when the expression profile of one or more genes and/or fragments thereof in the biological sample is either differentially expressed (over-expressed or under-expressed) or expressed at all when compared to the expression level of the corresponding gene and/or fragment thereof in the said control expression profile.

The authors of the present invention have found that from those listed genes in Figure 3, there are 122 differentially expressed genes in a pathological endometrium when compared to a healthy endometrium. In particular, it has been found that 59 genes are up-regulated and therefore, over-expressed and 63 genes are down regulated, and therefore under-expressed, as shown in Table 1 below, where the fold change (FC) and false discovery rate (FDR) show the factor of change of expression and the statistical significance of these changes of those genes respectively.

**Table 1: 122-Differentially expressed genes of the gene panel of Figure 3**

| **Gene** | **Gene description** | **FC** | **FDR** |
|---|---|---|---|
| **PAEP** | Progestagen Associated Endometrial Protein | 3.1873 | 9.12E-07 |
| **GPR110** | Adhesion G Protein-Coupled Receptor F1 | 2.3304 | 3.68E-05 |
| **CHST1** | Carbohydrate Sulfotransferase 1 | 2.0292 | 0.0004 |
| **POSTN** | Periostin | 1.8936 | 0.0009 |
| **BCL2L10** | BCL2 Like 10 | 1.8549 | 0.0001 |
| **TSPAN8** | Tetraspanin 8 | 1.8098 | 0.0179 |
| **CYP3A5** | Cytochrome P450 Family 3 Subfamily A Member 5 | 1.7966 | 0.0092 |
| **SFRP4** | Secreted Frizzled Related Protein 4 | 1.7778 | 1.23E-06 |
| **BAI2** | Adhesion G Protein-Coupled Receptor B2 | 1.7138 | 0.0018 |
| **ITGA8** | Integrin Subunit Alpha 8 | 1.7033 | 0.0005 |
| **PMEPA1** | Prostate Transmembrane Protein, Androgen Induced 1 | 1.7026 | 3.35E-07 |
| **CLU** | Clusterin | 1.6771 | 3.68E-05 |
| **MUC16** | Mucin 16, Cell Surface Associated | 1.6288 | 0.0001 |
| **MYH7B** | Myosin Heavy Chain 7B | 1.6034 | 0.0116 |
| **SCGB2A2** | Secretoglobin Family 2^{ª} Member 2 | 1.5859 | 0.0326 |
| **STC1** | Stannioclacin 1 | 1.5800 | 0.0033 |
| **FOXP1** | Forkhead Box P1 | 1.5332 | 3.94E-13 |
| **C10orf10** | DEPP1 Autophagy Regulator | 1.5093 | 0.0004 |
| **LRRC17** | Leucine Rich Repeat Containing 17 | 1.5020 | 0.0001 |
| **SERTAD4** | SERTA Domain Containing 4 | 1.4805 | 7.63E-07 |
| **COL16A1** | Collagen Type XVI Alpha 1 Chain | 1.4786 | 0.0024 |
| **C1orf133** | SERTAD4 Antisense RNA 1 | 1.4381 | 0.0065 |
| **MAPK8IP3** | Mitogen-Activated Protein Kinase 8 Interacting Protein 3 | 1.4275 | 0.0003 |
| **ABCC3** | ATP Binding Cassette Subfamily C Member 3 | 1.4181 | 0.0124 |
| **MFAP5** | Microfibril Associated Protein 5 | 1.4030 | 0.0033 |
| **MFAP2** | Microfibril Associated Protein 2 | 1.3990 | 0.0005 |
| **GPX3** | Glutathione Peroxidase 3 | 1.3960 | 0.0198 |
| **EDN3** | Endothelin 3 | 1.3627 | 0.0053 |
| **SORCS1** | Sortilin Related VPS10 Domain Containing Receptor 1 | 1.3563 | 0.0322 |
| **DUSP2** | Dual Specificity Phosphatase 2 | 1.3545 | 0.0264 |
| **SLPI** | Secretory Leukocyte Peptidase Inhibitor | 1.3463 | 0.0197 |
| **BICD1** | BICD Cargo Adaptor 1 | 1.3427 | 1.99E-05 |
| **CRABP2** | Cellular Retinoic Acid Binding Protein 2 | 1.3395 | 0.0040 |
| **ARID5B** | AT-Rich Interaction Domain 5B | 1.3247 | 1.31E-05 |
| **EVC** | EvC Ciliary Complex Subunit 1 | 1.3157 | 0.0011 |
| **C2CD4B** | C2 Calcium Dependent Domain Containing 4B | 1.3091 | 0.0109 |
| **ANO1** | Anoctamin 1 | 1.3014 | 0.0053 |
| **CLDN4** | Claudin 4 | 1.2867 | 0.0216 |
| **IGF2** | Insulin Like Growth Factor 2 | 1.2854 | 0.0134 |
| SYNE2 | Spectrin Repeat Containing Nuclear Envelope Protein 2 | 1.2756 | 5.71E-06 |
| **VAV3** | Vav Guanine Nucleotide Exchange Factor 3 | 1.2644 | 0.0286 |
| **ITGA9** | Integrin Subunit Alpha 9 | 1.2562 | 0.0084 |
| **OBFC2A** | Nucleic Acid Binding Protein 1 | 1.2266 | 0.0032 |
| **WEE1** | WEE1 G2 Checkpoint Kinase | 1.2190 | 6.54E-06 |
| **RAB40B** | RAB40B, Member RAS Oncogene Family | 1.2100 | 0.0042 |
| **BCL6** | BCL6 Transcription Repressor | 1.1891 | 0.0084 |
| **ID4** | Inhibitor Of DNA Binding 4, HLH Protein | 1.1878 | 0.0156 |
| **CD55** | CD55 Molecule (Cromer Blood Group) | 1.1806 | 0.0493 |
| **LETM2** | Leucine Zipper And EF-Hand Containing Transmembrane Protein 2 | 1.1754 | 0.0179 |
| **CYBRD1** | Cytochrome B Reductase 1 | 1.1647 | 0.0116 |
| **LIMK2** | LIM Domain Kinase 2 | 1.1614 | 0.0022 |
| **C14orf45** | Basal Body Orientation Factor 1 | 1.1526 | 0.0053 |
| **C16orf70** | Phagosome Assembly Factor 1 | 1.1514 | 0.0022 |
| **FOSL2** | FOS Like 2, AP-1 Transcription Factor Subunit | 1.1412 | 0.0193 |
| **SCAMP1** | Secretory Carrier Membrane Protein 1 | 1.1296 | 0.0264 |
| **CTDSP2** | CTD Small Phosphatase 2 | 1.1099 | 0.0166 |
| **RNF19A** | Ring Finger Protein 19A, RBR E3 Ubiquitin Protein Ligase | 1.1058 | 0.0250 |
| **HMGN3** | High Mobility Group Nucleosomal Binding Domain 3 | 1.0988 | 0.0286 |
| **ACTN1** | Actinin Alpha 1 | 1.0983 | 0.0322 |
| **ENY2** | ENY2 Transcription And Export Complex 2 Subunit | -1.0932 | 0.0466 |
| **PSMB10** | Proteasome 20S Subunit Beta 10 | -1.1231 | 0.0053 |
| **ARHGDIA** | Rho GDP Dissociation Inhibitor Alpha | -1.1245 | 0.0433 |
| **PRPF4** | Pre-MRNA Processing Factor 4 | -1.1397 | 0.0348 |
| **COPG1** | COPI Coat Complex Subunit Gamma 1 | -1.1433 | 0.0006 |
| **RACGAP1** | Rac GTPase Activating Protein 1 | -1.1496 | 0.0042 |
| **PNP** | Purine Nucleoside Phosphorylase | -1.1519 | 0.0143 |
| **STEAP4** | STEAP4 Metalloreductase | -1.1550 | 0.0270 |
| **BDH1** | 3-Hydroxybutyrate Dehydrogenase 1 | -1.1744 | 0.0030 |
| **ECI2** | Enoyl-CoA Delta Isomerase 2 | -1.1862 | 0.0178 |
| **ANK3** | Ankyrin 3 | -1.2084 | 0.0433 |
| **IL15** | Interleukin 15 | -1.2233 | 0.0198 |
| **GSN** | Gelsolin | -1.2241 | 0.0076 |
| **GLIPR1** | GLI Pathogenesis Related 1 | -1.2251 | 0.0166 |
| **CBR3** | Carbonyl Reductase 3 | -1.2252 | 0.0124 |
| **ATP6V0E2** | ATPase H+ Transporting V0 Subunit E2 | -1.2304 | 0.0082 |
| DYNLT3 | Dynein Light Chain Tctex-Type 3 | -1.2323 | 0.0199 |
| **S100A4** | S100 Calcium Binding Protein A4 | -1.2478 | 0.0041 |
| **BUB1B** | BUB1 Mitotic Checkpoint Serine/Threonine Kinase B | -1.2482 | 0.0451 |
| **IDH1** | Isocitrate Dehydrogenase (NADP(+)) 1 | -1.2488 | 0.0124 |
| **PLA2G16** | Phospholipase A And Acyltransferase 3 | -1.2498 | 0.0124 |
| **EPHB3** | EPH Receptor B3 | -1.2530 | 0.0396 |
| **OAZ3** | Ornithine Decarboxylase Antizyme 3 | -1.2581 | 0.0013 |
| **PYCR1** | Pyrroline-5-Carboxylate Reductase 1 | -1.2597 | 0.0007 |
| **FAM155B** | NALCN Channel Auxiliary Factor 2 | -1.2618 | 0.0116 |
| **ECHS1** | Enoyl-CoA Hydratase, Short Chain 1 | -1.2621 | 3.68E-05 |
| **CSRP2** | Cysteine And Glycine Rich Protein 2 | -1.2647 | 0.0023 |
| **TLR4** | Toll Like Receptor 4 | -1.2716 | 0.0065 |
| **CTSW** | Cathepsin W | -1.2764 | 0.0322 |
| **CEP55** | Centrosomal Protein 55 | -1.3021 | 0.0264 |
| **BARD1** | BRCA1 Associated RING Domain 1 | -1.3064 | 0.0014 |
| **PLA2G4A** | Phospholipase A2 Group IVA | -1.3101 | 0.0008 |
| **ATP1B1** | ATPase Na+/K+ Transporting Subunit Beta 1 | -1.3202 | 0.0113 |
| **FOXO1** | Forkhead Box O1 | -1.3330 | 0.0018 |
| **BIRC3** | Baculoviral IAP Repeat Containing 3 | -1.3496 | 0.0007 |
| **CRISP3** | Cysteine Rich Secretory Protein 3 | -1.3498 | 0.0397 |
| **LMCD1** | LIM And Cysteine Rich Domains 1 | -1.3627 | 0.0012 |
| **PSMB8** | Proteasome 20S Subunit Beta 8 | -1.3783 | 3.68E-05 |
| **CD81** | CD81 Molecule | -1.3888 | 0.0104 |
| **ALDH1A3** | Aldehyde Dehydrogenase 1 Family Member A3 | -1.3891 | 0.0007 |
| **SQLE** | Squalene Epoxidase | -1.3905 | 0.0001 |
| **KMO** | Kynurenine 3-Monooxygenase | -1.4107 | 0.0434 |
| **RPRM** | Reprimo, TP53 Dependent G2 Arrest Mediator Homolog | -1.4121 | 0.0061 |
| **SPP1** | Secreted Phosphoprotein 1 | -1.4130 | 0.0242 |
| **ANGPTL1** | Angiopoietin Like 1 | -1.4381 | 0.0017 |
| **HPRT1** | Hypoxanthine Phosphoribosyltransferase 1 | -1.4394 | 1.43E-05 |
| **ATP6V1A** | ATPase H+ Transporting V1 Subunit A | -1.4484 | 0.0040 |
| **CDK1** | Cyclin Dependent Kinase 1 | -1.4651 | 0.0084 |
| **ENPEP** | Glutamyl Aminopeptidase | -1.4704 | 0.0441 |
| **TH** | Tyrosine Hydroxylase | -1.5149 | 0.0113 |
| **G0S2** | G0/G1 Switch 2 | -1.5183 | 0.0226 |
| **PROS1** | Protein S | -1.5297 | 0.0025 |
| **STAR** | Steroidogenic Acute Regulatory Protein | -1.5342 | 0.0005 |
| **DKK1** | Dickkopf WNT Signaling Pathway Inhibitor 1 | -1.5425 | 0.0005 |
| **BANK1** | B Cell Scaffold Protein With Ankyrin Repeats 1 | -1.5553 | 0.0005 |
| **KCNJ2** | Potassium Inwardly Rectifying Channel Subfamily J Member 2 | -1.6602 | 0.0119 |
| **SLC7A2** | Solute Carrier Family 7 Member 2 | -1.6919 | 0.0038 |
| **PROK1** | Prokineticin 1 | -1.7218 | 0.0106 |
| **OLFM4** | Olfactomedin 4 | -1.7759 | 0.0458 |
| **IFNG** | Interferon Gamma | -1.9666 | 0.0033 |
| **CXCL13** | C-X-C Motif Chemokine Ligand 13 | -2.0690 | 0.0001 |
| **SLC16A6** | Solute Carrier Family 16 Member 6 | -2.2113 | 9.12E-07 |
| **FGB** | Fibrinogen Beta Chain | -2.3652 | 0.0108 |

Therefore, in a further embodiment, determining the risk of implantation failure may depend on determining over-expression of one or more genes and/or fragments thereof of Figure 3 in the biological sample isolated from a subject when compared to the expression level of the same genes and/or fragments thereof in the control expression profile.

In a further embodiment, determining the risk of implantation failure may depend on determining under-expression of one or more genes and/or fragments thereof of Figure 3 in the biological sample isolated from a subject when compared to the expression level of the same genes and/or fragments thereof in the control expression profile.

In a still further embodiment of the present invention, determining the risk of implantation failure in a subject may depend on determining under-expression of one or more genes and/or fragments thereof of Figure 3 in the biological sample isolated from the subject when compared to the expression level of the same genes and/or fragments thereof in the control expression profile and determining over-expression of one or more genes and/or fragments thereof of Figure 3 in the biological sample isolated from the subject when compared to the expression level of the same genes and/or fragments thereof in the control expression profile.

In addition, the authors of the present invention have found that from those listed genes in Figure 3, expression level of the following genes: SCGB3A1; DEGS1; SNX10; TFF3; SLC15A1; CYP2J2; MAP2K3; HPGD; CCBL1; MOCOS; ERP27; RARRES3 and XDH remain undetected in a healthy endometrium, whereas are detected, i.e., are shown to be expressed, in a pathological endometrium.

Thus, according to the present invention determining the risk of implantation failure may depend on determining at least expression of one or more genes and/or fragments thereof selected from SCGB3A1; DEGS1; SNX10; TFF3; SLC15A1; CYP2J2; MAP2K3; HPGD; CCBL1; MOCOS; ERP27; RARRES3 and XDH of Figure 3 in the biological sample isolated from a subject, which expression is undetectable in a control expression profile of a healthy endometrium.

Therefore, in a further embodiment of the present invention, determining the risk of implantation failure in a subject may depend on determining in the biological sample isolated from the subject, under-expression of one or more genes and/or fragments thereof of Figure 3 when compared to the expression level of the same genes and/or fragments thereof in the control expression profile of a healthy endometrium, determining over-expression of one or more genes and/or fragments thereof of Figure 3, when compared to the expression level of the same genes and/or fragments thereof in the control expression profile and determining expression of one or more genes and/or fragments thereof of Figure 3 in the biological sample isolated from the subject when expression of the same genes and/or fragments thereof in the control healthy expression profile are undetectable.

It follows the definition of the plurality of gene panels which according to the unexpected accuracy results shown in Example 2, serve for the purpose of the method of prognosing risk of IF in a subject disclosed in the present invention.

In a preferred embodiment the present invention is directed to an in vitro method for prognosing the risk of implantation failure of an embryo in a subject, comprising measuring in a biological sample isolated from said subject, the level of expression of a plurality of genes or fragments thereof of Figure 3, said plurality of genes comprising each of the following genes or fragments thereof as listed in Table 2 below:

**Table 2: 9-Gene panel and fragments thereof.**

| **Gene Name** | **Target Sequence** |
|---|---|
| ARID5B | SEQ ID NO. 10 |
| FOXP1 | SEQ ID NO. 80 |
| SFRP4 | SEQ ID NO. 152 |
| PMEPA1 | SEQ ID NO. 130 |
| PAEP | SEQ ID NO. 127 |
| SLC16A6 | SEQ ID NO. 154 |
| SERTAD4 | SEQ ID NO. 151 |
| WEE1 | SEQ ID NO. 177 |
| SYNE2 | SEQ ID NO. 169 |

The method further comprises comparing if each of said genes in the biological sample are differentially expressed with respect to a control expression profile (reference control) and determining risk of implantation failure in the subject if genes: PAEP; FOXP1; PMEPA1; ARID5B; SFRP4; SERTADA4; SYNE2 and WEE1; are over-expressed and gene: SLC16A6; is under-expressed when compared to the control (healthy) expression profile.

In a still preferred embodiment of the present invention the present invention's method for prognosing the risk of implantation failure of an embryo in a subject, comprises, in addition to measuring the level of expression of each of the genes or fragments thereof of Table 2, additionally measuring one or more of the following genes or fragments thereof as listed in Table 3 below:

**Table 3: 46-Gene panel and fragments thereof**

| **Gene Name** | **Target Sequence** |
|---|---|
| ALDH1A3 | SEQ ID NO. 5 |
| ANGPTL1 | SEQ ID NO. 6 |
| BAI2 | SEQ ID NO. 15 |
| BANK1 | SEQ ID NO. 16 |
| BARD1 | SEQ ID NO. 17 |
| BCL2L10 | SEQ ID NO. 21 |
| BDH1 | SEQ ID NO. 23 |
| BICD1 | SEQ ID NO. 24 |
| BIRC3 | SEQ ID NO. 25 |
| C100rf10 | SEQ ID NO. 27 |
| C16orf70 | SEQ ID NO. 30 |
| CLU | SEQ ID NO. 44 |
| ECHS1 | SEQ ID NO. 66 |
| FOXO1 | SEQ ID NO. 79 |
| DKK1 | SEQ ID NO. 63 |
| CSRP2 | SEQ ID NO. 51 |
| CXCL13 | SEQ ID NO. 55 |
| CRABP2 | SEQ ID NO. 47 |
| COPG1 | SEQ ID NO. 46 |
| EVC | SEQ ID NO. 74 |
| COL16A1 | SEQ ID NO. 45 |
| CHST1 | SEQ ID NO. 41 |
| GPR110 | SEQ ID NO. 84 |
| IFNG | SEQ ID NO. 99 |
| HPRT1 | SEQ ID NO. 96 |
| ITGA8 | SEQ ID NO. 103 |
| PROS1 | SEQ ID NO. 134 |
| PSMB8 | SEQ ID NO. 139 |
| PYCR1 | SEQ ID NO. 140 |
| PLA2G4A | SEQ ID NO. 129 |
| POSTN | SEQ ID NO. 132 |
| SQLE | SEQ ID NO. 161 |
| LIMK2 | SEQ ID NO. 110 |
| RACGAP1 | SEQ ID NO. 142 |
| OBFC2A | SEQ ID NO. 125 |
| LRRC17 | SEQ ID NO. 112 |
| MUC16 | SEQ ID NO. 122 |
| SLC7A2 | SEQ ID NO. 155 |
| MFAP2 | SEQ ID NO. 116 |
| OAZ3 | SEQ ID NO. 124 |
| MAPK8IP3 | SEQ ID NO. 114 |
| STC1 | SEQ ID NO. 163 |
| MFAP5 | SEQ ID NO. 118 |
| STAR | SEQ ID NO. 162 |
| RAB40B | SEQ ID NO. 141 |
| LMCD1 | SEQ ID NO. 111 |

The said method further comprising in addition to comparing expression of genes or fragments thereof of Table 2 as defined in preceding paragraphs, comparing if said one or more genes of Table 3 are differentially expressed with respect to the reference control profile, and lastly determining risk of implantation failure in the subject if additionally, said one or more of genes: EVC; STC1; MFAP5; OBFC2A; C16orf70; MUC16; ITGA8; CRABP2; POSTN; MFAP2; MAPK8IP3; RAB40B; LIMK2; BAI2; GPR110; COL16A1; CHST1; C10orf10; CLU; BICD1; BCL2L10 and LRRC17 are over-expressed and genes: BANK1; CXCL13; SQLE; FOXO1; PROS1; PSMB8; PLA2G4A; RACGAP1; BDH1; SLC7A2; ANGPTL1; ECHS1; OAZ3; HPRT1; COPG1; DKK1; LMCD1; PYCR1; IFNG; BIRC3; STAR; CSRP2; BARD1 and ALDH1A3 are under-expressed when compared to the reference control profile.

In a still preferred embodiment of the present invention the present invention's method for prognosing the risk of implantation failure of an embryo in a subject, comprises, in addition to measuring the level of expression of each of the genes or fragments thereof of Table 2 and one or more of the genes or fragments thereof as listed in Table 3, additionally measuring one or more of the following genes or fragments thereof as listed in Table 4 below:

**Table 4: 6-Gene panel and fragments thereof**

| **Gene Name** | **Target Sequence** |
|---|---|
| ANO1 | SEQ ID NO. 8 |
| ATP6V1A | SEQ ID NO. 14 |
| EDN3 | SEQ ID NO. 68 |
| PSMB10 | SEQ ID NO. 138 |
| S100A4 | SEQ ID NO. 146 |
| C14orf45 | SEQ ID No. 28 |

The said method further comprising in addition to comparing expression of genes or fragments thereof of Tables 2 and 3 as defined in preceding paragraphs, comparing if said one or more genes of Table 4 are differentially expressed with respect to the reference control profile, and lastly determining risk of implantation failure in the subject if additionally, said one or more of genes: C14orf45; ANO1 and EDN3 are over-expressed and/or said one or more of genes: S100A4; ATP6V1A and PSMB10 are under-expressed when compared to the reference control sample.

In a still preferred embodiment of the present invention the present invention's method for prognosing the risk of implantation failure of an embryo in a subject, comprises, in addition to measuring the level of expression of each of the genes or fragments thereof of Table 2 and one or more of the genes or fragments thereof as listed in Tables 3 and 4, additionally measuring one or more of the following genes or fragments thereof as listed in Table 5 below:

**Table 5: 14-Gene panel and fragments thereof**

| **Gene Name** | **Target Sequence** |
|---|---|
| ATP6V0E2 | SEQ ID NO. 12 |
| BCL6 | SEQ ID NO. 22 |
| C1orf133 | SEQ ID NO. 31 |
| C2CD4B | SEQ ID NO. 32 |
| CD81 | SEQ ID NO. 38 |
| CDK1 | SEQ ID NO. 39 |
| GSN | SEQ ID NO. 88 |
| CYP3A5 | SEQ ID NO. 5 |
| FGB | SEQ ID NO. 76 |
| CYBRD1 | SEQ ID NO. 56 |
| ITGA9 | SEQ ID NO. 104 |
| PROK1 | SEQ ID NO. 133 |
| RPRM | SEQ ID NO. 145 |
| TLR4 | SEQ ID NO. 172 |

The said method further comprising in addition to comparing expression of genes or fragments thereof of Tables 2, 3 and 4 as detailed in paragraphs above, comparing if said one or more genes or fragments thereof of Table 5 are differentially expressed with respect to the reference control profile, and lastly determining risk of implantation failure in the subject if additionally, said one or more of genes: C1orf133; BCL6; ITGA9; CYP3A5; C2CD4B and CYBRD1 are over-expressed and/or said one or more of genes: RPRM; TLR4; GSN; ATP6V0E2; CDK1; CD81 PROK1 and FGB are under-expressed when compared to the reference control sample.

In a still further preferred embodiment of the present invention the present invention's method for prognosing the risk of implantation failure of an embryo in a subject, comprises, in addition to measuring the level of expression of each of the genes or fragments thereof of Table 2 and one or more of the genes or fragments thereof as listed in Tables 3, 4 and 5, additionally measuring one or more of the following genes or fragments thereof as listed in Table 6 below:

**Table 6: 5-Gene panel and fragments thereof**

| **Gene Name** | **Target Sequence** |
|---|---|
| ATP1B1 | SEQ ID NO. 11 |
| CBR3 | SEQ ID NO. 33 |
| FAM155B | SEQ ID NO. 75 |
| MYH7B | SEQ ID NO. 123 |
| TH | SEQ ID NO. 171 |

The said method further comprising in addition to comparing expression of genes or fragments thereof of Tables 2, 3, 4 and 5 as detailed in paragraphs above, comparing if said one or more genes or fragments thereof of Table 6 are differentially expressed with respect to the reference control profile, and lastly determining risk of implantation failure in the subject if additionally, said one or more of genes: C1orf133; BCL6; ITGA9; CYP3A5; C2CD4B and CYBRD1 are over-expressed and/or said one or more of genes: RPRM; TLR4; GSN; ATP6V0E2; CDK1; CD81 PROK1 and FGB are under-expressed when compared to the reference control sample.

In a still further preferred embodiment of the present invention the present invention's method for prognosing the risk of implantation failure of an embryo in a subject, comprises, in addition to measuring the level of expression of each of the genes or fragments thereof of Table 2 and one or more of the genes or fragments thereof as listed in Tables 3, 4, 5 and 6, additionally measuring one or more of the following genes or fragments thereof as listed in Table 7 below:

**Table 7: 32-Gene panel and fragments thereof**

| **Gene Name** | **Target Sequence** |
|---|---|
| ABCC3 | SEQ ID NO. 1 |
| ACTN1 | SEQ ID NO. 4 |
| CTSW | SEQ ID NO. 54 |
| DUSP2 | SEQ ID NO. 64 |
| ECI2 | SEQ ID NO. 67 |
| CTDSP2 | SEQ ID NO. 53 |
| CEP55 | SEQ ID NO. 40 |
| FOSL2 | SEQ ID NO. 77 |
| GPX3 | SEQ ID NO. 86 |
| GLIPR1 | SEQ ID NO. 83 |
| DYNLT3 | SEQ ID NO. 65 |
| HMGN3 | SEQ ID NO. 91 |
| CLDN4 | SEQ ID NO. 42 |
| G0S2 | SEQ ID NO. 82 |
| KCNJ2 | SEQ ID NO. 106 |
| IDH1 | SEQ ID NO. 98 |
| IGF2 | SEQ ID NO. 100 |
| IL15 | SEQ ID NO. 101 |
| LETM2 | SEQ ID NO. 109 |
| ID4 | SEQ ID NO. 97 |
| PNP | SEQ ID NO. 131 |
| SPP1 | SEQ ID NO. 160 |
| SCAMP1 | SEQ ID NO. 148 |
| SORCS1 | SEQ ID NO. 159 |
| SCGB2A2 | SEQ ID NO. 149 |
| SLPI | SEQ ID NO. 157 |
| RNF19A | SEQ ID NO. 144 |
| PLA2G16 | SEQ ID NO. 128 |
| PRPF4 | SEQ ID NO. 135 |
| TSPAN8 | SEQ ID NO. 173 |
| STEAP4 | SEQ ID NO. 167 |
| VAV3 | SEQ ID NO. 175 |

The said method further comprising in addition to comparing expression of genes or fragments thereof of Tables 2, 3, 4, 5 and 6 as detailed in paragraphs above, comparing if said one or more genes or fragments thereof of Table 7 are differentially expressed with respect to the reference control profile, and lastly determining risk of implantation failure in the subject if additionally, said one or more of genes: ABCC3; CTDSP2; IGF2; ID4; LETM2; FOSL2; TSPAN8; SLPI, GPX3; RNF19A; CLDN4; SCAMP1; DUSP2; VAV3; SORCS1 ACTN1; HMGN3; and SCGB2A2, are over-expressed and/or said one or more of genes: KCNJ2; IDH1; PLA2G16; PNP, GLIPR1, ECI2; IL15; DYNLT3; G0S2; SPP1; STEAP4; CEP55; CTSW and PRPF4 are under-expressed when compared to the reference control sample.

In a still further preferred embodiment of the present invention the present invention's method for prognosing the risk of implantation failure of an embryo in a subject, comprises, in addition to measuring the level of expression of each of the genes or fragments thereof of Table 2 and one or more of the genes or fragments thereof as listed in Tables 3, 4, 5, 6 and 7, additionally measuring one or more of the following genes or fragments thereof as listed in Table 8 below:

**Table 8: 10-Gene panel and fragments thereof**

| **Gene Name** | **Target Sequence** |
|---|---|
| ANK3 | SEQ ID NO. 7 |
| ARHGDIA | SEQ ID NO. 9 |
| BUB1B | SEQ ID NO. 26 |
| CD55 | SEQ ID NO. 35 |
| EPHB3 | SEQ ID NO. 72 |
| ENPEP | SEQ ID NO. 69 |
| CRISP3 | SEQ ID NO. 49 |
| ENY2 | SEQ ID NO. 71 |
| KMO | SEQ ID NO. 107 |
| OLFM4 | SEQ ID NO. 126 |

The said method further comprising in addition to comparing expression of genes or fragments thereof of Tables 2, 3, 4, 5, 6 and 7 as detailed in paragraphs above, comparing if said one or more genes or fragments thereof of Table 8 are differentially expressed with respect to the reference control profile, and lastly determining risk of implantation failure in the subject if additionally, said one gene: CD55 is over-expressed and/or said one or more of genes: EPHB3; ARHGDIA; CRISP3; ENY2; ANK3; KMO; ENPEP; BUB1B and OLFM4 are under-expressed when compared to the reference control sample.

In a still further preferred embodiment of the present invention the present invention's method for prognosing the risk of implantation failure of an embryo in a subject, comprises, in addition to measuring the level of expression of each of the genes or fragments thereof of Table 2 and one or more of the genes or fragments thereof as listed in Tables 3, 4, 5, 6, 7 and 8, additionally measuring one or more of the following genes or fragments thereof as listed in Table 9 below:

**Table 9: 13-Gene panel and fragments thereof**

| **Gene Name** | **Target Sequence** |
|---|---|
| CCBL1 | SEQ ID NO. 34 |
| CYP2J2 | SEQ ID NO. 57 |
| HPGD | SEQ ID NO. 94 |
| ERP27 | SEQ ID NO. 73 |
| DEGS1 | SEQ ID NO. 59 |
| SLC15A1 | SEQ ID NO. 153 |
| RARRES3 | SEQ ID NO. 143 |
| MOCOS | SEQ ID NO. 121 |
| MAP2K3 | SEQ ID NO. 113 |
| SCGB3A1 | SEQ ID NO. 150 |
| SNX10 | SEQ ID NO. 158 |
| XDH | SEQ ID NO. 178 |
| TFF3 | SEQ ID NO. 170 |

The said method further comprising in addition to comparing expression of genes or fragments thereof of Tables 2, 3, 4, 5, 6, 7 and 8 as detailed in paragraphs above, comparing if said one or more genes or fragments thereof of Table 9 are expressed with respect to the reference control profile, and lastly determining risk of implantation failure in the subject if additionally, said one or more of genes: SCGB3A1; DEGS1; SNX10; TFF3; SLC15A1; CYP2J2; MAP2K3; HPGD; CCBL1; MOCOS; ERP27; RARRES3 and XDH are expressed when compared to the reference control sample, where expression of such genes of Table 9 is undetectable.

In a still particularly embodiment, the present invention is directed to a method for prognosing the risk of implantation failure of an embryo in a subject, which comprises measuring the level of expression of at least each of the genes of Figure 3; comparing if each of the genes or corresponding fragments thereof of Tables 2 to 8 are differentially expressed with respect to the reference control sample and comparing if each of the genes or fragments thereof of Table 9 are expressed with respect to the reference control sample, and lastly determining risk of implantation in the subject if each of the genes: EVC; STC1; MFAP5; OBFC2A; C16orf70; MUC16; ITGA8; PAEP; CRABP2; POSTN; MFAP2; MAPK8IP3; FOXP1; PMEPA1; BICD1; RAB40B; ARID5B; SFRP4; LIMK2; BAI2; GPR110; SERTADA4; COL16A1; SYNE2; CHST1; C10orf10; WEE1; CLU; BCL2L10; LRRC17; C14orf45; ANO1; EDN3; C1orf133; BCL6; ITGA9; CYP3A5; C2CD4B; CYBRD1; MYH7B; ABCC3; CTDSP2; IGF2; ID4; LETM2; FOSL2; TSPAN8; SLPI, GPX3; RNF19A; CLDN4; SCAMP1; DUSP2; VAV3, SORCS1; HMGN3; ACTN1; SCGB2A2 and CD55 are over-expressed and each of the genes: BANK1; CXCL13; SQLE; FOXO1; PROS1; PSMB8; PLA2G4A; SLC16A6; RACGAP1; BDH1; ECHS1; SLC7A2; ANGPTL1; OAZ3; COPG1; DKK1; LMCD1; PYCR1; IFNG; BIRC3; STAR; CSRP2; BARD1; ALDH1A3; HPRT1; S100A4; ATP6V1A; PSMB10; RPRM; TLR4; GSN; ATP6V0E2; CDK1; CD81; PROK1; FGB; ATP1B1; TH; FAM155B; CBR3; KCNJ2; IDH1; PLA2G16; PNP, GLIPR1, ECI2; IL15; DYNLT3; G0S2;; SPP1; STEAP4; CEP55;; CTSW; PRPF4; EPHB3; ARHGDIA; CRISP3; ENY2; ANK3; KMO; ENPEP; BUB1B and OLFM4 are under-expressed and each of the genes: SCGB3A1; DEGS1; SNX10; TFF3; SLC15A1; CYP2J2; MAP2K3; HPGD; CCBL1; MOCOS; ERP27; RARRES3 and XDH are expressed in the biological sample when compared to the reference control sample, wherein expression of such genes of Table 9 is undetectable.

In a further embodiment of the present invention the methods described herein are directed to determining the risk of implantation failure of an embryo in an endometrium by identifying and determining the expression level of genes or fragments thereof that are related to a possible pathology of the endometrium, rather than genes that measure the endometrial progression effect.

Thus the present invention is also directed to a method for obtaining an endometrial pathology gene panel for use in a gene signature discovery of an endometrium being at risk of having an IF. The process of obtaining such endometrial pathology gene panel comprises removing the gene expression data corresponding to variations in the timing of the window of implantation (WOI) of genes of Table 10 for removing the menstrual cycle progression effect from the endometrial pathology gene panel. Such endometrial pathology gene panel (EP) is useful in the definition of the predictive gene panels of Tables 2 to 9 of the present invention

The correction for the said endometrial progression effect, uses the ED model described before that is based on machine learning algorithmsand the gene expression data of the 73 genes or fragments thereof of Table 10 below (ED signature). First subjects are classified in the different menstrual cycle phases using ED model and then the menstrual cycle effect is removed as explained before.

**Table 10: 73-Gene ED signature**

| **Gene Name** | **Target Sequence** |
|---|---|
| MAOA | SEQ ID NO. 2 |
| CREB3L1 | SEQ ID NO. 3 |
| C4BPA | SEQ ID NO. 13 |
| CD55 | SEQ ID NO. 35 |
| STC1 | SEQ ID NO. 163 |
| GALNT12 | SEQ ID NO. 18 |
| PELI1 | SEQ ID NO. 19 |
| FAM59A | SEQ ID NO. 20 |
| SLC16A6 | SEQ ID NO. 154 |
| GPX3 | SEQ ID NO. 86 |
| MFAP5 | SEQ ID NO. 118 |
| TTC21B | SEQ ID NO. 29 |
| CBR3 | SEQ ID NO. 33 |
| MAP2K6 | SEQ ID NO. 36 |
| ECM1 | SEQ ID NO. 37 |
| EDN3 | SEQ ID NO. 68 |
| ENY2 | SEQ ID NO. 71 |
| SCGB2A2 | SEQ ID NO. 149 |
| RASSF2 | SEQ ID NO. 43 |
| NFIA | SEQ ID NO. 48 |
| PRC1 | SEQ ID NO. 50 |
| ALPL | SEQ ID NO. 52 |
| EFNA1 | SEQ ID NO. 60 |
| SOX17 | SEQ ID NO. 61 |
| CDC20 | SEQ ID NO. 62 |
| PAEP | SEQ ID NO. 127 |
| NNMT | SEQ ID NO. 70 |
| TRIM22 | SEQ ID NO. 78 |
| GABARAPL1 | SEQ ID NO. 81 |
| ECI2 | SEQ ID NO. 67 |
| LRP4 | SEQ ID NO. 85 |
| CP | SEQ ID NO. 87 |
| KCNG1 | SEQ ID NO. 89 |
| PTPRR | SEQ ID NO. 90 |
| NR4A2 | SEQ ID NO. 92 |
| SOD2 | SEQ ID NO. 93 |
| OFD1 | SEQ ID NO. 95 |
| CYP3A5 | SEQ ID NO. 58 |
| OLFM4 | SEQ ID NO. 126 |
| ARG2 | SEQ ID NO. 102 |
| MPPED2 | SEQ ID NO. 105 |
| ABCC3 | SEQ ID NO. 1 |
| CKB | SEQ ID NO. 108 |
| BIRC3 | SEQ ID NO. 25 |
| OPRK1 | SEQ ID NO. 115 |
| EPHB3 | SEQ ID NO. 72 |
| HABP2 | SEQ ID NO. 117 |
| PRKCQ | SEQ ID NO. 119 |
| SLC7A1 | SEQ ID NO. 120 |
| IDH1 | SEQ ID NO. 98 |
| KMO | SEQ ID NO. 107 |
| ATP6V0E2 | SEQ ID NO. 12 |
| STEAP4 | SEQ ID NO. 167 |
| ARID5B | SEQ ID NO. 10 |
| HPSE | SEQ ID NO. 136 |
| HAL | SEQ ID NO. 137 |
| CSRP2 | SEQ ID NO. 51 |
| CALB2 | SEQ ID NO. 147 |
| SYNE2 | SEQ ID NO. 169 |
| TSPAN8 | SEQ ID NO. 173 |
| SPDEF | SEQ ID NO. 156 |
| SLC15A1 | SEQ ID NO. 153 |
| CYP2J2 | SEQ ID NO. 57 |
| IER3 | SEQ ID NO. 164 |
| MSX1 | SEQ ID NO. 165 |
| DEFB1 | SEQ ID NO. 166 |
| S100P | SEQ ID NO. 168 |
| ID4 | SEQ ID NO. 97 |
| C2CD4B | SEQ ID NO. 32 |
| C100rf10 | SEQ ID NO. 27 |
| PAQR4 | SEQ ID NO. 174 |
| GREM2 | SEQ ID NO. 176 |
| HPRT1 | SEQ ID NO. 96 |

The effect of menstrual cycle progression may be removed from gene expression data using an R/Bioconductor software package that provides an integrated solution for analyzing data from gene expression experiments, for example, the removeBatchEffect function based on linear models implemented in the *limma* R package v.3.30.13, because this function enables correcting the bias from a known batch effect (e.g., menstrual cycle) while indicating the group differences to be retained in the transcriptomic data (e.g., risk of implantation failure due to endometrial pathology versus control).

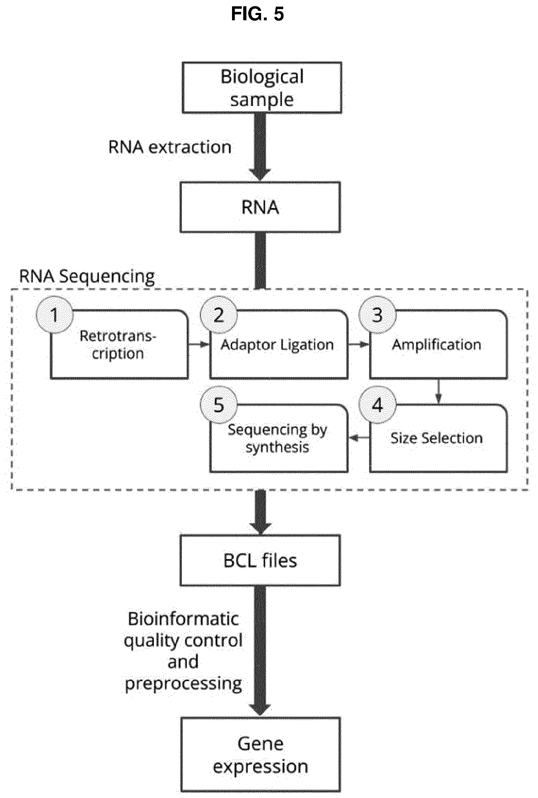

The biological sample used according to the method herein described is preferably an endometrial biopsy sample of a human female, from which RNA can be extracted to determine expression level of the gene panels identified in preceding paragraphs. RNA extraction and RNA sequencing procedure is followed as illustrated in Figure 5.

The expression level of a gene in the gene population of the present invention can be determined by a variety of methods, including, but not limited to, detecting the amount of nucleic acid of the gene and the polypeptide encoded thereby. The skilled person can select the type and amount of sample as required, and select the conventional techniques in the art to perform the determination of gene expression level.

Alternatively, determining the expression level of a gene panel is carried out by detecting the amount of the polypeptide encoded by the corresponding genes. The detection can be accomplished by using commonly known reagents with techniques known in the art, including, but not limited to, enzyme-linked immunosorbent assay (ELISA), chemiluminescent immunoassay techniques (e.g., immunochemiluminometric assay, chemiluminescent enzyme immunoassay, electrochemiluminescent immunoassay), flow cytometry, Immunohistochemistry (IHC).

Preferably, the methods herein described detect the amount of nucleic acid of the genes. The detection may be accomplished by commonly known reagents and techniques known in the art, including, but not limited to, molecular hybridization techniques (microarrays), quantitative PCR techniques, or nucleic acid sequencing techniques (RNAseq), among others. Molecular hybridization techniques include, but are not limited to, ISH techniques (e.g., DISH, DNA-FISH, RNA-FISH, CISH techniques, etc.), DNA or RNA imprinting techniques, gene chip techniques (e.g., microarray or microfluidic chip techniques), and the like, preferably in situ hybridization techniques. Quantitative PCR techniques include, but are not limited to, semi-quantitative PCR and RT-PCR techniques, preferably RT-PCR techniques. Nucleic acid sequencing techniques include RNA-sequencing techniques, but are not limited to, Sanger sequencing, Next Generation Sequencing (NGS), third generation sequencing, single cell sequencing techniques, and the like.

Preferably, determining the expression level of the genes in the gene panels herein described is determined using a next generation sequencing technique.

The mRNA/protein expression can be determined by known methods, e.g., in situ hybridization, reverse transcription PCR, immunohistochemistry and RNA sequencing. Methods for quantifying mRNA are well known in the art. For example, mRNA can be quantified by isolating RNA from a biological sample (e.g., endometrial biopsy sample) from a patient. General methods for mRNA extraction are well known in the art. The extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous. Next generation sequencing techniques selected from RNA-sequencing techniques, quantitative PCR and/or PCR or gene expression microarrays are being more preferred.

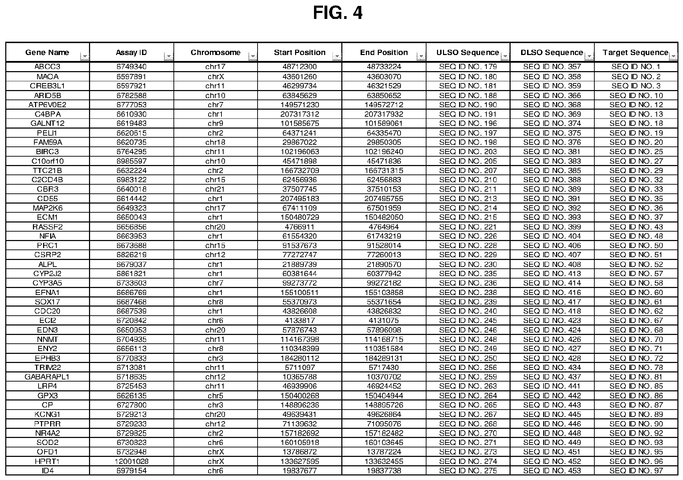

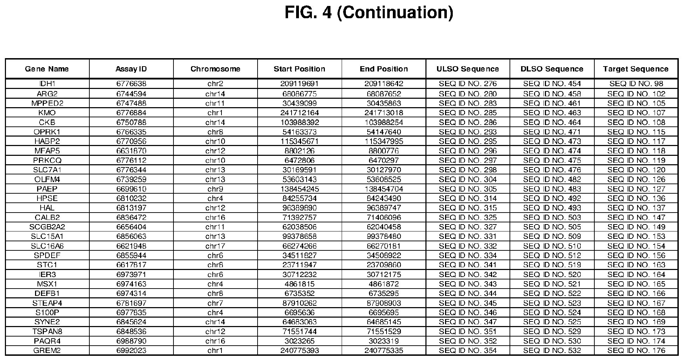

Generally, the first step in gene expression profiling by RT-PCR is the reverse transcription of the RNA template into cDNA, followed by its exponential amplification in a PCR reaction, by contacting the generated cDNA with pairs of primers targeting the genes, as listed in Figures 3 and 4. Thereafter gene expression data is collected using real-time PCR and subsequently analyzed by means of commonly used software-extracting total RNA in the sample. And finally determining the risk of IF including RIF by subjecting the obtained sequencing results to statistical analysis.

A preferred procedure (Figure 5) for RNA sequencing comprises the steps of:
**1. RNA extraction:** total RNA or a specific type of RNA is extracted from biological sample and is or not fragmented depending on the protocol. There are several protocols to extract RNA in the laboratory and commercial kits. Necessary RNA input will depend on the Illumina protocol.
**2. Retrotranscription:** RNA extracted is retrotranscribed to cDNA through the action of retrotranscriptases.
**3. Adaptor ligation:** some adaptors are ligated to 3' and 5' extremes from cDNA fragments. These adaptors include sequences employed to identify the origin sample of the fragments called barcodes or indexes like single index or unique dual indexes (i5 and i7), but also sequences that allow to amplify them or link them to sequencing support like primer binding sites (P5 or P7).
**4. Amplification:** cDNA fragments with adaptors are amplified by PCR more or less depending on the Illumina protocol.
**5. Size selection:** a size selection (150-200bp) is carried out during bead-based library purification.
**6. Sequencing:** once finished the library generation process, a sequencing step is required. Concretely, Illumina systems employ sequencing by synthesis (SBS).
**7. BCL files:** these files are generated with the sequencing reads after sequencing.
**8. Gene expression:** a set of bioinformatic tools provide filtered reads after quality control and a preprocessing step for normalising the gene expression data ready for downstream analysis.

### Generation of the prediction model:

The present disclosure also discloses a method for generating a prediction model for carrying out the prognostic method of the invention. This method of generating a prediction model uses at least one algorithm for analyses of gene expression data to generate the prediction model from a library of known profile patterns. Such a system for analysis of data to generate a prediction model and validation thereof comprises: a computing environment; an input device, connected to the computing environment, to receive data from a user, wherein the data received comprises the items of information from the human female subject to provide the profile for said female subject, an output device, connected to the computing environment, to provide information to the user; and a computer readable storage medium having stored thereon at least one algorithm to generate the prediction model by identifying the prognostic variables that are predictive of the subject being at risk of IF, including RIF, wherein the system provides results that can be used for generation of a report providing prognostic results of that subject suffering IF, including RIF.

In particular, for the generation of the prediction model of the present disclosure, a new stratification methodology was used, referred herein as "Clinically Acute Transcriptomic Stratification" (CATranS), (Figure 7) as it uses an initial acute clinical classification of patients as RIF or control and then stratifies patients by their endometrial transcriptome to pathological or healthy profiles. These two groups have been confirmed to show differential reproductive outcomes in the next single embryo transfer after biopsy collection, with a significantly different pregnancy rate. In particular, the authors of the present invention have found that these two groups (Figures 8A & 8B) had a significantly different pregnancy rate (45.1% vs 79.6%, pathological vs healthy, respectively, p-value=3.8E-5), live birth rate (56.4% vs 97.5%, respectively, p-value=3.0E-06), clinical pregnancy rate (27.9% vs 2.6%, respectively, p-value=0.0020), and biochemical pregnancy rate (20.4% vs 0%, respectively, p-value=0.0023). Comparing the two groups, it has been found that there were at least 122 differentially expressed genes (FDR<0.05) in endometrial pathology, 59 up- and 63 downregulated, as well as at least additionally 13 expressed genes in endometrial pathology, which were not expressed in the healthy endometrium, i.e., expression of those genes was undetectable in a healthy endometrium. This gene signature could most preferably be used as a signature discovery for prognosing the risk of implantation failure of an embryo in an endometrium.

Based on the above related findings, both of methods, tests and kits for prognosing the risk of IF of an embryo in an endometrium, due to pathology of the said endometrium, have been disclosed for the first time in the present invention. Said methods comprising identification of expression of genes and/or specific relevant regions in said genes and corresponding kits for performing these methods providing the expected prognostic effect with a high and reliable accuracy.

Thus, the present invention discloses a new methodology for obtaining a prediction model for stratifying subjects as being at a risk of implantation failure of an embryo in the endometrium. Said new methodology comprises the steps of (Figure 6)
(1) Endometrial Pathology (EP) gene expression dataset comprising 404 genes selected to be related to endometrial dating (ED panel) and/or pathological recurrent implantation failure (RIF) conditions (obtained from GSE58144 dataset analysis) was applied in 217 patients with long-term clinical follow-up. (See Figure 6)
(2) CATranS methodology, as shown in Figure 7, was defined and applied to the EP dataset. Briefly, acute clinical criteria were used to propose an initial extreme classification of patients into two conditions to: healthy endometrium and pathological endometrium. Patients not meeting the acute clinical criteria were classified as "non-labelled." Machine learning was supervised by pathological and healthy categories from CATranS taxonomy. Iterations of the above defined methodology reclassified samples according to their probability of belonging to a determined class according to their transcriptomics profiles and evaluated the improvement in terms of number of differential expressed genes obtained with any new reclassification of samples until the algorithm converged on the optimal solution.
(3) CATranS methodology evaluation. Stratification obtained from the methodology above was evaluated in terms of improvement in molecular differences and in clinical outcomes between transcriptomic profiles. (Figures 8). Pathological patient profile identified with the method of the present invention had from 1.5 till 3.3-times higher relative risk than healthy patients of not achieving full-term pregnancy.
(4) Prediction performance evaluation. A gene signature discovery (Figure 2) was made using the whole set of classified data. Using this signature, prediction performance was evaluated through a 5-fold cross-validation process repeated 100 times, (Figure 9). Prediction performance for the gene signatures identified resulted in at median accuracy of 0.92, a median sensitivity of 0.96, and mean median specificity of 0.84. Following the CATranS methodology referred above, the present invention discloses for a first time several gene signatures which a high and reliable accuracy for predicting IF, including RIF not caused by asynchronies in menstrual cycle progression, but due to a pathological endometrium condition.

Thus, the method of the present invention for prognosing risk of IF overcomes misclassifications due to unknown clinical variables for classifying patients into two groups by leveraging a transcriptomic identifier of endometrial pathology independent of the health record, and thus prevents the loss of good-quality embryos in in vitro fertilization (IVF) treatments.

### Accuracy of the method of invention

Accuracy for the proposed method was by following a 5-fold cross-validation process (Figure 9) repeated 100 times using both 135-gene signature described in Figure 3 and support vector machine (SVM) machine learning models. For each cross-validation process, subjects are randomly divided in 5 sets and on each fold, four of the sets were used to train the model and the remaining set was used to validate the model and calculate the prediction performance of the model (accuracy) relative to each fold. Then the final accuracy of the cross validation process is the mean of the accuracies obtained on each fold. With this process, all subjects in the complete set of data were used four times to train the model and one to validate it. This cross-validation process is repeated 100 times and the final accuracy provided is the mean of the 100 cross-validation processes performed.

### Kits:

The present disclosure also provides a kit for practicing the prognostic method of the present invention. The kit may include a carrier for the various components of the kit. The kit many include oligonucleotides specifically hybridizing under high stringency to mRNA or cDNA of the genes and fragments thereof as listed in Figure 3 and Figure 4. Such oligonucleotides can be used as PCR primers in RT-PCR reactions, or hybridization probes. In some embodiments the kit comprises reagents (e.g., probes, primers, and or antibodies) for determining the expression level of the specific panel of genes. The oligonucleotides in the detection kit can be labeled with any suitable detection marker including but not limited to, radioactive isotopes, fluorophores, biotin, enzymes (e.g. alkaline phosphatase), enzyme substrates, ligands and antibodies. The kit can also comprise any other commonly used components useful in the detection techniques described above. Examples of such components include, but are not limited to, Taq polymerase, deoxyribonucleotides, dideoxyribonucleotides, other primers suitable for the amplification of a target DNA sequence, RNase A, and the like. In addition, the detection kit preferably includes instructions on using the kit for practice the prognostic method of the present invention.

### EXAMPLES

### Example 1: Construction of a prediction model

### Study design

A customized RNA-seq panel ("Endometrial Pathology" or "EP" panel) encompassing 404 markers of endometrial progression and potential pathologies (as listed in Figure 11) were used to measure gene expression in 217 patients. This EP panel was created combining information from GSE58144 data set from GEO and the ED panel. The EP panel gene expression data were used to develop a new methodology for stratification of subjects, as a basis for defining a method of prognosing risk of IF, including RIF. This methodology was called Clinical Acute Transcriptomics Stratification (CATranS) (Figure 7). This procedure allowed to stratify the population as having pathological or healthy endometrium according to both clinical characteristics and transcriptomic profiles. To evaluate the benefits of this proposed stratification (Figure 7), the molecular and reproductive outcome differences between stratified groups was analyzed. CATranS classification was used to supervise machine learning algorithms and to build a prototype that was assessed as a diagnostic tool by calculating prediction performance parameters. This new genomic tool provided a promising model for preventively evaluating the WOI to distinguish endometrial pathology independent of endometrial progression (Figure 7).

### Endometrial Pathology gene panel

Gene selection for the EP panel is detailed in Figure 6. First, a whole-transcriptome endometrial dataset (GSE58144, n=115) from GEO (https://www.ncbi.nlm.nih.gov/geo/) was used to identify genes potentially relevant for RIF diagnosis. GSE58144 dataset was downloaded from GEO and pre-processed as described before. Briefly, data was downloaded and normalized using standardized methods. Then machine learning models were applied to create a transcriptomic-based taxonomy that classify samples regarding both endometrial progression and endometrial disruption This transcriptomic-based taxonomy was used to correct the endometrial progression effect. A pathological gene signature discovery process was performed to find genes related to transcriptomic-based pathological profiles (endometrial disruption profile). Additionally, genes prioritized for endometrial dating prediction previously (ERA^{®}, WIN^{®} and ERmap^{®}) as well as a set of 50 genes selected as optimal signature (Figure 2) from 4 whole-transcriptome datasets for endometrial dating (GSE98386, GSE29981, GSE58144 and GSE4888) were included in the EP panel (as listed in Figure 11) to properly correct endometrial progression bias, as a necessary step to unmask pathology

### Endometrial biopsies

This prospective, multicenter study was conducted between July 2018 and July 2021 at five reproductive medicine clinics from the same provider system in Spain. Patients invited to participate were scheduled for endometrial evaluation for medical decision or due to ≥2 implantation failures of idiopathic origin and met the following inclusion criteria: age 18-50 years, body mass index (BMI) of 19-30 kg/m2, undergoing a hormone replacement therapy (HRT) cycle, endometrial thickness >6.5 mm with trilaminar structure, and serum levels of estradiol (E2) >100 pg/mL and progesterone (P4) <1 ng/mL on the 10th day of estrogen treatment.

Endometrial biopsies were obtained from uterine fundus after approximately 120 hours of progesterone administration as part of the HRT cycle. After RNA extraction, only samples with RNA input of 200 ng, 260/280 ratio of ~2, 260/230 ratio of 1.8-2.2, and percentage of RNA fragments larger than 200 nucleotides (DV200) ≥70% were sequenced.

### Endometrial sequencing and transcriptomic pre-processing

The EP dataset included endometrial RNA samples targeted for sequencing the EP gene panel using a TruSeq Targeted RNA Illumina protocol (https://www.illumina.com/products/by-type/sequencing-kits/library-prep-kits/truseq-targeted-rna.html, Illumina). Samples were sequenced in a NextSeq 550 with a pairedend design of 150 cycles and 4 million reads/sample. Raw data were evaluated using FastQC tool for read quality. STAR was employed for mapping quality data, using as reference the gene sequences included in the designed panel. Raw counts were obtained using featureCounts, which calculates parameters such as Phred quality score (Q). Low-quality raw counts (Q < 30) were filtered. In addition, low-read samples (<2M) were filtered. Remaining data were normalized by calculating the normalization factors, filtering low-expressed genes and using Voom transformation and quantile normalization included in limma R-package. Then, menstrual cycle effect was removed on endometrial gene expression using linear models included in limma R-package. This correction method was supervised by transcriptomic endometrial dating provided by the classification of ED model described previously. This correction procedure ensures the evaluation of endometrial pathology that would be confounded by transcriptome changes related to endometrial progression.

### Development of a transcriptomic stratification procedure for pathological endometria: CATranS

A new stratification procedure was developed based on semi-supervised classification methods. It refers to a classification that involves a small portion of labelled examples and a large number of unlabelled examples from which a model must learn and make predictions on new examples. We termed this stratification procedure Clinical Acute Transcriptomics Stratification (CATranS), as it uses an initial acute clinical classification of patients as RIF or control and then stratifies patients by their endometrial transcriptome to pathological or healthy profiles.

The CATranS procedure comprises the following iterative steps (Figure 7):
1. Initial clinical-based acute classification (Figure 7.1): Patients were initially classified as RIF or control based on available clinical information on number of IVF cycles performed at the recruiting clinic and clinical outcome for each single embryo transferred. In this procedure, clinically acute RIF (aRIF) and control (aFertile) groups were defined to extremes, and this clinical-based acute classification was set as the "current" classification. Differential expression analysis (DEA) was applied between aRIF and aFertile groups (limma R-package) to evaluate if the molecular differences between groups increase or not across the iterations Finally, the EP gene expression was analyzed for a signature discovery to distinguish between the two groups. This signature was used to train all successive classification models.
2. Multiclassifier method (Figure 7.2): Supervised by the current classification of samples and using the gene signature obtained in step 1, support vector machine (SVM) and random forest (RF) probabilistic models were trained using R package RWeka.
3. Additive approach (Figure 7.3): The whole sample set was tested using both models (SVM and RF) trained in the previous step. Probability of being pathological was defined for each patient as the average probability of being pathological in SVM and RF models. This mean probability (p) obtained for each patient was used to reclassify them, so a patient was newly classified as having a pathological endometrium if p>0.75 and was newly classified as having a healthy endometrium if p<0.25. Samples with 0.25<p<0.75 were considered as not properly recognized by the model.
4. Stopping criteria (Figure 7.4): First, if any sample was reclassified in a different category from the "current" classification, this classification was considered the optimal classification according to transcriptomic profile. If any sample was reclassified, a DEA was applied between pathological and healthy endometrial groups according to the new proposed classification. Significant differential expressed genes (DEGs) were identified as those having an adjusted p-value by false discovery rate (FDR) lower than 0.05. If the number of DEGs obtained was equal to or greater than that obtained with the current classification, the new classification was set as the new current classification, and the algorithm returned to step 2. If the number of DEGs obtained was less than before, the new proposed classification was discarded, and the previous classification was set as the optimal classification of samples according to their transcriptomic profile.

### Characterization of differentially expressed genes

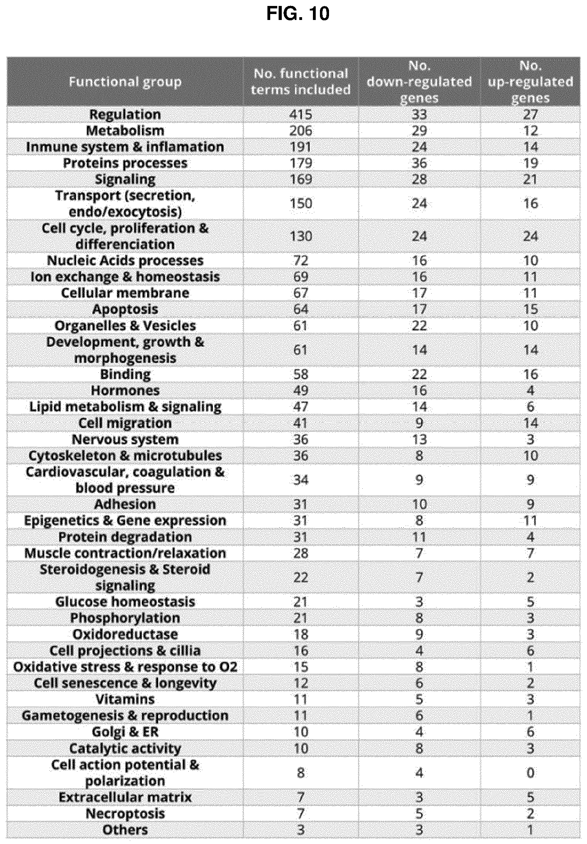

Obtained DEGs were functionally annotated for interpretation by consulting Kyoto Encyclopedia of Genes and Genomes (KEGG) human pathways (release 99.0, August 1, 2021) and Gene Ontology (GO) (Biological Process, Molecular Function and Cellular Component) (version 2021-07-02, propagated annotation); only experimental annotations were kept based on GO guidelines. Functional terms were manually grouped in 19 functional categories according to functional description of each term in the database (Figure 10).

### Evaluation of clinical reproductive outcomes

Pregnancy rate (PR) was defined as the proportion of positive pregnancy tests obtained at the first transfer after biopsy collection in the patient population undergoing embryo transfer; live birth rate was defined as the number of live births divided by the total number of positive pregnancy tests obtained at the first transfer after biopsy collection. Clinical pregnancy rate was defined as the proportion of clinically recognized pregnancy loss before the 20th week of gestation related to the total number of pregnancies in which a gestational sac was visualized, and biochemical pregnancy rate was defined as the proportion of gestational losses after a positive pregnancy test without visualization of the gestational sac. Differences in proportions between considered groups on each comparison were statistically tested using Fisher's exact test (Figure 8).

### Prediction performance calculation by balanced prediction model cross-validation

Transcriptomic data were reduced to samples previously transcriptomic-based classified as pathological or healthy with a probability of >0.75 for pathology or <0.25 for healthy endometria. Signature discovery based on support vector machine (SVM) models was performed to distinguish between pathological and healthy samples classified according to CATranS. Then, a 5-fold 100-times cross-validation procedure (Figure 9) based on SVM models included in WEKA was performed to obtain the range of classification performance parameters: accuracy, sensitivity, and sensibility.

### Statistical analysis

Experimental and clinical characteristics of patients included in pathological and healthy groups are described using mean and standard deviation for continuous variables and counts and percentage for discrete variables. These characteristics were compared between groups using Wilcoxon rank-sum test for continuous variables and Fisher's exact test for discrete variables.

### Example 2: Accuracy of Gene panels

Genes were ordered according to their prediction capability from best to worst. Accuracy for each proposed set of genes was calculated (defined in Tables 1-10) by following a 5-fold cross-validation process (Figure 9) repeated 100 times. For each cross-validation process, subjects are randomly divided into 5 sets and on each fold, four of the sets were used to train the model and the remaining set was used to validate the model and calculate the prediction performance of the model (accuracy) relative to each fold. Then the final accuracy of the cross-validation process is the mean of the accuracies obtained on each fold. With this process, all subjects in the complete set of data were used four times to train the model and one to validate it. This cross-validation process is repeated 100 times and the final accuracy provided for each proposed set of genes is the mean of the 100 cross-validation processes performed.

Results are shown in the following Table 12.

**Table: 12: Accuracy of gene panels according to the present invention**

| **Gene Panel** | **Sensitivity** | **Specificity** | **Accuracy** |
|---|---|---|---|
| Table 2 | 0.9113 | 0.7547 | 0.88 |
| Table 2 +Table 3 | 0.9343 | 0.8367 | 0.9086 |
| Table 2+Table 3+ S100A4 | 0.9416 | 0.8367 | 0.9140 |
| Table 2+Table 3+ S100A4 + ATP6V1A + PSMB10 +C14orf45 | 0.9343 | 0.8367 | 0.9086 |
| Table 2+Table 3+Table 4 | 0.9416 | 0.8367 | 0.9086 |
| Table 2+Table 3+Table 4 + Table 5 | 0.9489 | 0.8367 | 0.9194 |
| Table 2+Table 3+Table 4 + Table 5 + ATP1 B1+TH | 0.9562 | 0.8367 | 0.9194 |
| Table 2+Table 3+Table 4 + Table 5 + ATP1B1+TH + FAM155B | 0.9562 | 0.8367 | 0.9194 |
| Table 2+Table 3+Table 4+Table 5+ Table 6 | 0.9489 | 0.8367 | 0.9194 |
| Table 2+Table 3+Table 4+Table 5+ Table 6 + CEP55 | 0.9489 | 0.8367 | 0.9140 |
| Table 2+Table 3+Table 4+Table 5+ Table 6 + CEP55 + DUSP2 | 0.9489 | 0.8367 | 0.9140 |
| Table 2+Table 3+Table 4+Table 5+ Table 6 + CEP55 + DUSP2+ ACTN1 | 0.9489 | 0.8367 | 0.9140 |
| Table 2+Table 3+Table 4+Table 5+ Table 6 + CEP55 + DUSP2+ ACTN1 +VAV3 | 0.9489 | 0.8367 | 0.9194 |
| Table 2+Table 3+Table 4+Table 5+ Table 6 + CEP55 + DUSP2+ ACTN1 +VAV3 + CTSW | 0.9489 | 0.8367 | 0.9194 |
| Table 2+Table 3+Table 4+Table 5+ Table 6 + CEP55 + DUSP2+ ACTN1 +VAV3 + CTSW + SORCS1 | 0.9489 | 0.8367 | 0.9194 |
| Table 2+Table 3+Table 4+Table 5+ Table 6+ CEP55 + DUSP2+ ACTN1 +VAV3 + CTSW + SORCS1 + PRPF4 | 0.9489 | 0.8367 | 0.9194 |
| Table 2+Table 3+Table 4+Table 5+ Table 6 + Table 7 | 0.9489 | 0.8367 | 0.9140 |
| Table 2+Table 3+Table 4+Table 5+ Table 6 + Table 7 + Table 8 | 0.9562 | 0.8367 | 0.9194 |
| Table 2+Table 3+Table 4+Table 5+ Table 6 + Table 7 + Table 8 + Table 9 | 0.9562 | 0.8367 | 0.9194 |

**Table: 13: Accuracy of a gene panel of the prior art associated with genes measuring the endometrial progression effect**

| **Gene Panel** | **Sensitivity** | **Specificity** | **Accuracy** |
|---|---|---|---|
| Table 10 | 0.907 | 0.5510 | 0.8306 |

As can be seen from the results provided in Tables 12 and 13, gene panels used in a method for predicting IF according to the present invention as shown in Table 12 show an unexpectedly improved accuracy of at least 0.88 when compared to the use in the same prediction method of a gene panel according to state of the art, which measures only endometrial progression corresponding to variations in the timing of the window of implantation (WOI), and achieves an accuracy of 0.83. In addition, the gene panel used in the prior art shows a specificity of only 0.55 whereas the specificity achieved by the method of prediction of the present invention using gene panels of Table 10, is of at least 0.8367. Thus, the present invention method shows a significant higher discriminative power than when using gene signatures based on endometrial dating, as has been reported so far for both commercially available tests as well as scientific literature.

### References:

Altmäe, S., Koel, M., Võsa, U., Adler, P., Suhorutsenko, M., Laisk-Podar, T., Kukushkina, V., Saare, M., Velthut-Meikas, A., Krjutskov, K., Aghajanova, L., Lalitkumar, P. G., Gemzell-Danielsson, K., Giudice, L., & Simón, C. (2017). Meta-signature of human endometrial receptivity: a meta- analysis and validation study of transcriptomic biomarkers. Scientific Reports, 7, 10077. https://doi.org/10.1038/s41598-017-10098-3
Altmäe, S., Martínez-Conejero, J. A., Salumets, A., Simón, C., Horcajadas, J. A., & Stavreus-Evers, A. (2009). Endometrial gene expression analysis at the time of embryo implantation in women with unexplained infertility. Molecular Human Reproduction, 16(3), 178-187. https://doi.org/10.1093/molehr/gap102
Bastu, E., Demiral, I., Gunel, T., Ulgen, E., Gumusoglu, E., Kazem Hosseini, M., Sezerman, U., Buyru, F., & Yeh, J. (2019). Potential Marker Pathways in the Endometrium That May Cause Recurrent Implantation Failure. Reprod. Sci., 26(7), 879-890.
Bersinger, N. A., Wunder, D. M., Birkhäuser, M. H., & Mueller, M. D. (2008). Gene expression in cultured endometrium from women with different outcomes following IVF. Molecular Human Reproduction, 14(8), 475-484. https://doi.org/10.1093/molehr/gan036
Bhagwat, S. R., Chandrashekar, D. S., Kakar, R., Davuluri, S., Bajpai, A. K., Nayak, S., Bhutada, S., Acharya, K., & Sachdeva, G. (2013). Endometrial receptivity: a revisit to functional genomics studies on human endometrium and creation of HGEx-ERdb. PLoS One, 8(3), e58419.
Borthwick, J. M., Charnock-Jones, D. S., Tom, B. D., Hull, M. L., Teirney, R., Phillips, S. C., & Smith, S. K. (2003). Determination of the transcript profile of human endometrium. Molecular Human Reproduction, 9(1), 19-33. https://doi.org/10.1093/molehr/gag004
Busnelli, A., Reschini, M., Cardellicchio, L., Vegetti, W., Somigliana, E., & Vercellini, P. (2020). How common is real repeated implantation failure? An indirect estimate of the prevalence. Reproductive BioMedicine Online, 40(1), 91-97. https://doi.org/10.1016/j.rbmo.2019.10.014
Carson, D. D., Lagow, E., Thathiah, A., Al-Shami, R., Farach-Carson, M. C., Vernon, M., Yuan, L., Fritz, M. A., & Lessey, B. (2002). Changes in gene expression during the early to mid-luteal (receptive phase) transition in human endometrium detected by high-density microarray screening. Molecular Human Reproduction, 8(9), 871-879. https://doi.org/10.1093/molehr/8.9.871
Coughlan, C., Ledger, W., Wang, Q., Liu, F., Demirol, A., Gurgan, T., Cutting, R., Ong, K., Sallam, H., & Li, T. C. (2014). Recurrent implantation failure: Definition and management. In Reproductive BioMedicine Online. https://doi.org/10.1016/j.rbmo.2013.08.011
Coutifaris, C., Myers, E. R., Guzick, D. S., Diamond, M. P., Carson, S. A., Legro, R. S., McGovern, P. G., Schlaff, W. D., Carr, B. R., Steinkampf, M. P., Silva, S., Vogel, D. L., & Leppert, P. C. (2004). Histological dating of timed endometrial biopsy tissue is not related to fertility status. Fertility and Sterility, 82(5), 1264-1272. https://doi.org/10.1016/J.FERTNSTERT.2004.03.069
Craciunas, L., Gallos, I., Chu, J., Bourne, T., Quenby, S., Brosens, J. J., & Coomarasamy, A. (2019). Conventional and modern markers of endometrial receptivity: a systematic review and meta-analysis. Human Reproduction Update, 25(2), 202-223. https://doi.org/10.1093/humupd/dmy044
Devesa-Peiro, A., Sebastian-Leon, P., Pellicer, A., & Diaz-Gimeno, P. (2021). Guidelines for biomarker discovery in endometrium: Correcting for menstrual cycle bias reveals new genes associated with uterine disorders. Molecular Human Reproduction, 27(4), 1-19. https://doi.org/10.1093/molehr/gaab011
Díaz-Gimeno, P., Horcajadas, J. A., Martínez-Conejero, J. A., Esteban, F. J., Alamá, P., Pellicer, A., & Simón, C. (2011). A genomic diagnostic tool for human endometrial receptivity based on the transcriptomic signature. Fertility and Sterility, 95(1), 50-60.e15. https://doi.org/10.1016/j.fertnstert.2010.04.063
Díaz-Gimeno, P., Ruíz-Alonso, M., Blesa, D., & Simón, C. (2014). Transcriptomics of the human endometrium. International Journal of Developmental Biology, 58(2-4), 127-137. https://doi.org/10.1387/ijdb.130340pd
Díaz-Gimeno, P., Ruiz-Alonso, M., Sebastian-Leon, P., Pellicer, A., Valbuena, D., & Simón, C. (2017). Window of implantation transcriptomic stratification reveals different endometrial subsignatures associated with live birth and biochemical pregnancy. Fertility and Sterility, 108(4). https://doi.org/10.1016/j.fertnstert.2017.07.007
Diaz-Gimeno, P., Sebastian-Leon, P., Sanchez-Reyes, J. M., Spath, K., Aleman, A., Vidal, C., Devesa-Peiro, A., Labarta, E., Sánchez-Ribas, I., Ferrando, M., Kohls, G., García-Velasco, J. A., Seli, E., Wells, D., & Pellicer, A. (2022). Identifying and optimizing human endometrial gene expression signatures for endometrial dating. Human Reproduction, 37(2), 284-296. https://doi.org/10.1093/HUMREP/DEAB262
Enciso, M., Carrascosa, J. P., Sarasa, J., Martínez-Ortiz, P. A., Munné, S., Horcajadas, J. A., & Aizpurua, J. (2018). Development of a new comprehensive and reliable endometrial receptivity map (ER Map/ER Grade) based on RT-qPCR gene expression analysis. Human Reproduction, 33(2), 220-228. https://doi.org/10.1093/HUMREP/DEX370
Haouzi, D., Mahmoud, K., Fourar, M., Bendhaou, K., Dechaud, H., De Vos, J., Reme, T., & Dewailly, D.Hamamah, S. (2009). Identification of new biomarkers of human endometrial receptivity in the natural cycle. Human Reproduction, 24(1), 198-205. https://doi.org/10.1093/humrep/den360
Kao, L. C. (2002). Global Gene Profiling in Human Endometrium during the Window of Implantation. Endocrinology, 143(6), 2119-2138. https://doi.org/10.1210/en.143.6.2119
Koler, M., Achache, H., Tsafrir, A., Smith, Y., Revel, A., & Reich, R. (2009). Disrupted gene pattern in patients with repeated in vitro fertilization (IVF) failure. Human Reproduction, 24(10), 2541-2548. https://doi.org/10.1093/humrep/dep193
Koot, Y. E. M. M., van Hooff, S. R., Boomsma, C. M., Van Leenen, D., Groot Koerkamp, M. J. A., Goddijn, M., Eijkemans, M. J. C. C., Fauser, B. C. J. M. J. M., Holstege, F. C. P. P., Macklon, N. S., Koerkamp, M. J. A. G., Goddijn, M., Eijkemans, M. J. C. C., Fauser, B. C. J. M. J. M., Holstege, F. C. P. P., & Macklon, N. S. (2016). An endometrial gene expression signature accurately predicts recurrent implantation failure after IVF. Scientific Reports, 6(1), 19411. https://doi.org/10.1038/srep19411
Mirkin, S., Arslan, M., Churikov, D., Corica, A., Diaz, J. I., Williams, S., Bocca, S., & Oehninger, S. (2005). In search of candidate genes critically expressed in the human endometrium during the window of implantation. Human Reproduction, 20(8), 2104-2117. https://doi.org/10.1093/humrep/dei051
Mirnezami, R., Nicholson, J., & Darzi, A. (2012). Preparing for precision medicine. The New England Journal of Medicine, 366(6). https://doi.org/10.1056/NEJMP1114866
Murray, M. J., Meyer, W. R., Zaino, R. J., Lessey, B. A., Novotny, D. B., Ireland, K., Zeng, D., & Fritz, M. A. (2004). A critical analysis of the accuracy, reproducibility, and clinical utility of histologic endometrial dating in fertile women. Fertility and Sterility, 81(5), 1333-1343. https://doi.org/10.1016/J.FERTNSTERT.2003.11.030
Noyes, R. W., Hertig, A. T., & Rock, J. (1975). Dating the endometrial biopsy. American Journal of Obstetrics & Gynecology, 122(2), 262-263. https://doi.org/10.1016/S0002-9378(16)33500-1
Pathare, A. D. S., Zaveri, K., & Hinduja, I. (2017). Downregulation of genes related to immune and inflammatory response in IVF implantation failure cases under controlled ovarian stimulation. American Journal of Reproductive Immunology, 78(1), 1-13. https://doi.org/10.1111/aji.12679
Pirtea, P., De Ziegler, D., Tao, X., Sun, L., Zhan, Y., Ayoubi, J. M., Seli, E., Franasiak, J. M., & Scott, R. T. (2021). Rate of true recurrent implantation failure is low: results of three successive frozen euploid single embryo transfers. Fertility and Sterility, 115(1), 45-53. https://doi.org/10.1016/j.fertnstert.2020.07.002
Polanski, L. T., Baumgarten, M. N., Quenby, S., Brosens, J., Campbell, B. K., & Raine-Fenning, N. J. (2014). What exactly do we mean by "recurrent implantation failure"? A systematic review and opinion. Reproductive BioMedicine Online, 28(4), 409-423. https://doi.org/10.1016/j.rbmo.2013.12.006
Ponnampalam, A. P., Weston, G. C., Trajstman, A. C., Susil, B., & Rogers, P. A. W. (2004). Molecular classification of human endometrial cycle stages by transcriptional profiling. Molecular Human Reproduction, 10(12), 879-893. https://doi.org/10.1093/molehr/gah121
Punyadeera, C., Dassen, H., Klomp, J., Dunselman, G., Kamps, R., Dijcks, F., Ederveen, A., De Goeij, A., & Groothuis, P. (2005). Oestrogen-modulated gene expression in the human endometrium. Cellular and Molecular Life Sciences, 62(2), 239-250. https://doi.org/10.1007/s00018-004-4435-y
Riesewijk, A., Martin, J., van Os, R., Horcajadas, J. A., Polman, J., Pellicer, A., Mosselman, S., & Simón, C. (2003). Gene expression profiling of human endometrial receptivity on days LH+2 versus LH+7 by microarray technology. Molecular Human Reproduction, 9(5-6), 253-264. https://doi.org/10.1093/molehr/gag037
Sandra, O. (2016). Hormonal control of implantation. Annales d'Endocrinologie, 77(2), 63-66. https://doi.org/10.1016/j.ando.2016.04.013
Sebastian-Leon, P., Devesa-Peiro, A., Aleman, A., Parraga-Leo, A., Arnau, V., Pellicer, A., & Diaz-Gimeno, P. (2021). Transcriptional changes through menstrual cycle reveal a global transcriptional derepression underlying the molecular mechanism involved in the window of implantation. Molecular Human Reproduction, 27(5), 1-16. https://doi.org/10.1093/molehr/gaab027
Sebastian-Leon, P., Garrido, N., Remohí, J., Pellicer, A., & Diaz-Gimeno, P. (2018). Asynchronous and pathological windows of implantation: Two causes of recurrent implantation failure. Human Reproduction, 33(4), 626-635. https://doi.org/10.1093/humrep/dey023
Shi, C., Han, H. J., Fan, L. J., Guan, J., Zheng, X. B., Chen, X., Liang, R., Zhang, X. W., Sun, K. K., Cui, Q. H., & Shen, H. (2018). Diverse endometrial mRNA signatures during the window of implantation in patients with repeated implantation failure. Human Fertility, 21(3), 183-194. https://doi.org/10.1080/14647273.2017.1324180
Sigurgeirsson, B., Åmark, H., Jemt, A., Ujvari, D., Westgren, M., Lundeberg, J., & Gidlöf, S. (2017). Comprehensive RNA sequencing of healthy human endometrium at two time points of the menstrual cycle. Biology of Reproduction, 96(1), 24-33. https://doi.org/10.1095/BIOLREPROD.116.142547
Sun, Y., Zhang, Y., Ma, X., Jia, W., & Su, Y. (2021). Determining Diagnostic Criteria of Unexplained Recurrent Implantation Failure: A Retrospective Study of Two vs Three or More Implantation Failure. Frontiers in Endocrinology, 12(July), 1-8. https://doi.org/10.3389/fendo.2021.619437
Talbi, S., Hamilton, A. E., Vo, K. C., Tulac, S., Overgaard, M. T., Dosiou, C., Le Shay, N., Nezhat, C. N., Kempson, R., Lessey, B. A., Nayak, N. R., & Giudice, L. C. (2006). Molecular phenotyping of human endometrium distinguishes menstrual cycle phases and underlying biological processes in normo-ovulatory women. Endocrinology, 147(3), 1097-1121. https://doi.org/10.1210/en.2005-1076
Tapia, A., Gangi, L. M., Zegers-Hochschild, F., Balmaceda, J., Pommer, R., Trejo, L., Pacheco, I. M., Salvatierra, A. M., Henríquez, S., Quezada, M., Vargas, M., Ríos, M., Munroe, D. J., Croxatto, H. B., & Velasquez, L. (2008). Differences in the endometrial transcript profile during the receptive period between women who were refractory to implantation and those who achieved pregnancy. Human Reproduction, 23(2), 340-351. https://doi.org/10.1093/humrep/dem319
Wilcox, A. J., Baird, D. D., & Weinberg, C. R. (1999). Time of Implantation of the Conceptus and Loss of Pregnancy. Obstetrical & Gynecological Survey, 54(11), 705. https://doi.org/10.1097/00006254-199911000-00018

## Claims

1. - An in vitro method for prognosing the risk of implantation failure of an embryo in a subject, said implantation failure being due to molecular disruption of endometrium, comprising:
a) Measuring the level of expression of at least each of the genes of Table 2; in a biological sample isolated from said subject,
b) Comparing if each of said genes in the biological sample are differentially expressed with respect to a reference control sample, and
c) Determining risk of implantation failure in the subject if genes: PAEP; FOXP1; PMEPA1; ARID5B; SFRP4; SERTADA4; SYNE2 and WEE1; are over-expressed and gene: SLC16A6; is under-expressed when compared to the reference control sample.

2. - Method according to claim 1, which additionally comprises in step a) measuring the level of expression of one or more genes of Table 3 in step b) additionally comparing if said one or more genes of Table 3 are differentially expressed with respect to the reference control sample, and in step c) determining risk of implantation failure in the subject if additionally, said one or more of genes: EVC; STC1; MFAP5; OBFC2A; C16orf70; MUC16; ITGA8; CRABP2; POSTN; MFAP2; MAPK8IP3; RAB40B; LIMK2; BAI2; GPR110; COL16A1; CHST1; C10orf10; CLU; BICD1; BCL2L10 and LRRC17 are over-expressed and genes : BANK1; CXCL13; SQLE; FOXO1; PROS1; PSMB8; PLA2G4A; RACGAP1; BDH1; SLC7A2; ANGPTL1; ECHS1; OAZ3; HPRT1; COPG1; DKK1; LMCD1; PYCR1; IFNG; BIRC3; STAR; CSRP2; BARD1 and ALDH1A3 are under-expressed when compared to the reference control sample.

3. - Method according to claim 2, which additionally comprises in step a) measuring the level of expression of one or more of genes of Table 4; in step b) additionally comparing if said one or more of the genes of Table 4 are differentially expressed with respect to the reference control sample, and in step c) determining risk of implantation failure in the subject if additionally, said one or more of genes: C14orf45; ANO1 and EDN3 are over-expressed and/or said one or more of genes: S100A4; ATP6V1A and PSMB10 are under-expressed when compared to the reference control sample.

4. Method according to claim 3, which additionally comprises in step a) measuring the level of expression of one or more of genes of Table 5 in step b) additionally comparing if said one or more of the genes of Table 5 are differentially expressed with respect to the reference control sample, and in step c) determining risk of implantation failure in the subject if additionally, said one or more of genes: C1orf133; BCL6; ITGA9; CYP3A5; C2CD4B and CYBRD1 are over-expressed and/or said one or more of genes: RPRM; TLR4; GSN; ATP6V0E2; CDK1; CD81 PROK1 and FGB are under-expressed when compared to the reference control sample.

5. Method according to claim 4, which additionally comprises in step a) measuring the level of expression of one or more of genes of Table 6; in step b) additionally comparing if said one or more of the genes of Table 6 are differentially expressed with respect to the reference control sample, and in step c) determining risk of implantation failure in the subject if additionally, gene: MYH7B is over-expressed and/or said one or more of genes: ATP1B1; TH; FAM155B and CBR3 are under-expressed when compared to the reference control sample.

6. Method according to claim 5, which additionally comprises in step a) measuring the level of expression of one or more of genes of Table 7; in step b) additionally comparing if said one or more of the genes of Table 7 are differentially expressed with respect to the reference control sample, and in step c) determining risk of implantation failure in the subject if additionally, said one or more of genes: ABCC3; CTDSP2; IGF2; ID4; LETM2; FOSL2; TSPAN8; SLPI, GPX3; RNF19A; CLDN4; SCAMP1; DUSP2; VAV3; SORCS1 ACTN1; HMGN3; and SCGB2A2, are over-expressed and/or said one or more of genes: KCNJ2; IDH1; PLA2G16; PNP, GLIPR1, ECI2; IL15; DYNLT3; G0S2; SPP1; STEAP4; CEP55; CTSW and PRPF4 are under-expressed when compared to the reference control sample.

7. Method according to claim 6, which additionally comprises in step a) measuring the level of expression of one or more of genes of Table 8; in step b) additionally comparing if said one or more of the genes of Table 8 are differentially expressed with respect to the reference control sample, and in step c) determining risk of implantation failure in the subject if additionally, said one gene: CD55 is over-expressed and/or said one or more of genes: EPHB3; ARHGDIA; CRISP3; ENY2; ANK3; KMO; ENPEP; BUB1B and OLFM4 are under-expressed when compared to the reference control sample.

8. Method according to claim 7, which additionally comprises in step a) measuring the level of expression of one or more of genes of Table 9; in step b) additionally comparing if said one or more of the genes of Table 9 are expressed with respect to the reference control sample, and in step c) determining risk of implantation failure in the subject if additionally, said one or more of genes: SCGB3A1; DEGS1; SNX10; TFF3; SLC15A1; CYP2J2; MAP2K3; HPGD; CCBL1; MOCOS; ERP27; RARRES3 and XDH are expressed when compared to the reference control sample.

9. Method according to claim 8 which comprises in step a) measuring the level of expression of at least each of the genes of Figure 3; in step b) comparing if each of the genes of Tables 2 to 8 are differentially expressed with respect to the reference control sample and comparing if each of the genes of Table 9 are expressed with respect to the reference control sample, and in step c) determining risk of implantation in the subject if each of the genes: EVC; STC1; MFAP5; OBFC2A; C16orf70; MUC16; ITGA8; PAEP; CRABP2; POSTN; MFAP2; MAPK8IP3; FOXP1; PMEPA1; BICD1; RAB40B; ARID5B; SFRP4; LIMK2; BAI2; GPR110; SERTADA4; COL16A1; SYNE2; CHST1; C10orf10; WEE1; CLU; BCL2L10; LRRC17; C14orf45; ANO1; EDN3; C1orf133; BCL6; ITGA9; CYP3A5; C2CD4B; CYBRD1; MYH7B; ABCC3; CTDSP2; IGF2; ID4; LETM2; FOSL2; TSPAN8; SLPI, GPX3; RNF19A; CLDN4; SCAMP1; DUSP2; VAV3, SORCS1; HMGN3; ACTN1; SCGB2A2 and CD55 are over-expressed and each of the genes: BANK1; CXCL13; SQLE; FOXO1; PROS1; PSMB8; PLA2G4A; SLC16A6; RACGAP1; BDH1; ECHS1; SLC7A2; ANGPTL1; OAZ3; COPG1; DKK1; LMCD1; PYCR1; IFNG; BIRC3; STAR; CSRP2; BARD1; ALDH1A3; HPRT1; S100A4; ATP6V1A; PSMB10; RPRM; TLR4; GSN; ATP6V0E2; CDK1; CD81; PROK1; FGB; ATP1B1; TH; FAM155B; CBR3; KCNJ2; IDH1; PLA2G16; PNP, GLIPR1, ECl2; IL15; DYNLT3; G0S2;; SPP1; STEAP4; CEP55;; CTSW; PRPF4; EPHB3; ARHGDIA; CRISP3; ENY2; ANK3; KMO; ENPEP; BUB1B and OLFM4 are under-expressed and each of the genes: SCGB3A1; DEGS1; SNX10; TFF3; SLC15A1; CYP2J2; MAP2K3; HPGD; CCBL1; MOCOS; ERP27; RARRES3 and XDH are expressed in the biological sample when compared to the reference control sample.

10. Method according to any preceding claim, wherein the biological sample is an endometrial biopsy or uterine fluid sample.

11. Method according to any preceding claim, wherein determining risk of implantation failure comprises determining a higher than 1,5 relative risk of implantation failure not achieving a full-term pregnancy when compared to a reference control sample of a healthy subject.

12. Method according to any preceding claim, wherein the subject is a primate or a human female.

13. Method according to claim 11, wherein the endometrial biopsy has been taken in the mid secretory phase after a hormonal replacement therapy with estrogens and progesterone, in an artificial natural cycle inducing the ovulation with hCG or in a natural cycle.

14. Method according to any preceding claim, wherein measuring the level of expression of genes is performed through determining mRNA expression by Next Generation Sequencing (NGS) techniques, selected from: RNA-sequencing techniques, quantitative PCR and/or realtime PCR or gene expression microarrays.

15. Kit for use in an in vitro method for prognosing the risk of implantation failure of an embryo in a subject, according to any of claims 1 to 14, comprising reagents for detecting the expression level of genes of Tables 2 to 9.

16. A computer program product comprising instructions which, when executed by a computer, causes the computer to perform the method according to any of claims 1 to 14.

17. Method for obtaining an endometrial pathology gene panel for gene signature discovery of an endometrium being at risk of implantation failure (IF), including recurrent implantation failure (RIF), which comprises removing the gene expression data corresponding to variations in the timing of the window of implantation (WOI) of genes of Table 10 for removing the menstrual cycle progression effect from the endometrial pathology gene panel.
